# EUROPEAN PATENT SPECIFICATION

(11) **EP 1 969 371 B1**
(45) Date of publication and mention of the grant of the patent: **16.05.2012**
(21) Application number: 06843125.3
(22) Date of filing: 19.12.2006
(51) Int. Cl.: G01N 33/543, G01N 33/68

(54) **A METHOD OF PRODUCING A MULTIMERIC CAPTURE AGENT FOR BINDING A LIGAND**
VERFAHREN ZUR HERSTELLUNG EINES MULTIMEREN EINFANGMITTELS ZUR BINDUNG EINES LIGANDEN
PROCÉDÉ DESTINÉ À LA PRODUCTION D'UN AGENT DE CAPTURE MULTIMÈRE SERVANT À LIER UN LIGAND

(30) Priority: 20.12.2005 GB 0525918
(43) Date of publication of application: 17.09.2008
(73) Proprietor: Sharp Kabushiki Kaisha, Osaka-shi, Osaka 545-8522 (JP)
(72) Inventor: ANDERSON, Sally, Oxford OX2 6RQ (GB); REEVE, Michael, A., Henley-on-Thames, Oxfordshire RG9 1PL (GB)
(74) Representative: Baldock, Sharon Claire
(86) International application number: PCT/JP2006/325698
(87) International publication number: WO 2007/072976

(56) References cited:
- EP-A- 1 969 369
- WO-A-02/064619
- WO-A-2005/014615
- WO-A1-00/52456
- WO-A2-2004/058946
- JP-A- 03 083 600
- JP-A- 2000 510 233
- JP-A- 2001 131 208
- JP-A- 2003 284 552
- JP-A- 2005 504 309
- JP-A- 2005 516 595
- JP-A- 2005 524 057
- US-A- 5 491 074
- US-A- 5 750 332
- DATABASE WPI Week 199432 Thomson Scientific, London, GB; AN 1994-260510 XP002518999 & JP 06 192290 A (KURARAY CO LTD) 12 July 1994 (1994-07-12)
- ZHULI WAN ET AL: "Enhancing the Activity of Insulin at the Receptor Interface: Crystal Structure and Photo-Cross-Linking of A8 Analogues", BIOCHEMISTRY, AMERICAN CHEMICAL SOCIETY, US, vol. 43, no. 51, 1 January 2004 (2004-01-01), pages 16119-16133, XP007914939, ISSN: 0006-2960, DOI: DOI:10.1021/BI048223F [retrieved on 2004-11-25]
- KAZUNORI MATSUURA ET AL: "Artificial Peptide-Nanospheres Self-Assembled from Three-Way Junctions of alpha-Sheet-Forming Peptides", JOURNAL OF THE AMERICAN CHEMICAL SOCIETY, AMERICAN CHEMICAL SOCIETY, US, vol. 127, 1 January 2005 (2005-01-01), pages 10148-10149, XP007916431, ISSN: 0002-7863, DOI: DOI:10.1021/JA052644I [retrieved on 2005-06-30]
- CHERONIS J C ET AL: "A new class of bradykinin antagonists: synthesis and in vitro activity of bissuccinimidoalkane peptide dimers", JOURNAL OF MEDICINAL CHEMISTRY, AMERICAN CHEMICAL SOCIETY, US, vol. 35, no. 9, 1 May 1992 (1992-05-01), pages 1563-1572, XP002246182, ISSN: 0022-2623, DOI: 10.1021/JM00087A010

### SEQUENCE LISTING

<110> Sharp Kabushiki Kaisha
<120> A METHOD 0F PRODUCING A MULTIMERIC CAPTURE AGENT FOR BINDING A LIGAND
<130> 05R00965
<150> GB 0525918.9
   <151> 2005-12-20
<160> 66
<170> Patent In version 3.3
<210> 1
   <211> 13
   <212> PRT
   <213> Artificial Sequence
<220>
   <223> Synthetic Construct
<220>
   <221> BLINDING
   <222> (6).. (6)
   <223> Ligand binding residue
<220>
   <221> BINDING
   <222> (8)..(8)
   <223> Ligand binding residue
<220>
   <221> BINDING
   <222> (10).. (10)
   <223> Ligand binding residue
<400> 1
   Phe Gly Phe Cys Phe Xaa Phe Xaa Phe Xaa Phe Gly Phe
   1               5                   10
<210> 2
   <211> 7
   <212> PRT
   <213> Artificial Sequence
<220>
   <223> Synthetic Construct
<220>
   <221> SITE
   <222> (1).. (1)
   <223> Cysteine with activated thiol
<220>
   <221> BINDING
   <222> (2).. (2)
   <223> Ligand binding residue
<220>
   <221> BINDING
   <222> (4) .. (4)
   <223>. Ligand binding residue
<220>
   <221> BINDING
   <222> (6)..(6)
   <223> Ligand binding residue
<400> 2
   Xaa Xaa Phe Xaa Phe Xaa Phe
   1               5
<210> 3
   <211> 9
   <212> PRT
   <213> Artificial sequence
<220>
   <223> Synthetic Construct
<220>
   <221> SITE
   <222> (1.).. (1)
   <223> Norleucine
<220>
   <221> SITE
   <222> (3)..(3)
   <223> Norleucine
<220>
   <221> SITE
   <222> (5)..(5)
   <223> Norleucine
<220>
   <221> SITE
   <222> (7)..(7).
   <223> Norleucine
<220>
   <221> SITE
   <222> (9)..(9)
   <223> Norleucine
<400> 3
   Xaa Asp Xaa Ala Xaa Ser Xaa Lys Xaa
   1               5
<210> 4
   <211> 9
   <212> PRT
   <213> Artificial sequence
<220>
   <223> Synthetic Construct
<400> 4
   Phe Asp Phe Ala Phe Ser Phe Lys Phe
   1               5
<210> 5
   <211> 9
   <212> PRT
   <213> Artificial sequence
<220>
   <223> Synthetic Construct
<400> 5
   Ser Asp Ser Ala Ser Ser Ser Lys Ser
   1               5
<210> 6
   <211> 9
   <212> PRT
   <213> Artificial sequence
<220>
   <223> Synthetic Construct
<400> 6
   Asp Asp Asp Ala Asp Ser Asp Lys Asp
   1 5
<210> 7
   <211> 7
   <212> PRT
   <213> Artificial sequence
<220>
   <223> Synthetic Construct
<220>
   <221> SITE
   <222> (2)..(2)
   <223> Norleucine
<220>
   <221> SITE
   <222> (4)..(4)
   <223> Norleucine
<220>
   <221> SITE
   <222> (6)...(6)
   <223> Norleucine
<400> 7
   Asp Xaa Ala Xaa Ser Xaa Lys
   1 5
<210> 8
   <211> 7
   <212> PRT
   <213> Artificial sequence
<220>
   <223> Synthetic Construct
<400> 8
   Asp Phe Ala Phe Ser Phe Lys
   1 5
<210> 9
   <211> 7
   <212> PRT
   <213> Artificial sequence
<220>
   <223> Synthetic Construct
<400> 9
   Asp Ser Ala Ser Ser Ser Lys
   1               5
<210> 10
   <211> 7
   <212> PRT
   <213> Artificial sequence
<220>
   <223> Synthetic Construct
<400> 10
   Asp Asp Ala Asp Ser Asp Lys
   1               5
<210> 11
   <211> 9
   <212> PRT
   <213> Artificial sequence
<220>
   <223> Synthetic Construct
<220>
   <221> SITE
   <222> (1)..(1)
   <223> Norleucine
<220>
   <221> SITE.
   <222> (3).. (3)
   <223> Norleucine
<220>
   <221> SITE .
   <222> (5)..(5)
   <223> Norleucine
<220>
   <221> SITE
   <222> (6)..(6)
   <223> Norleucine
<220> <221> misc_feature
   <222> (7)..(7)
   <223> Xaa can be any naturally occurring amino acid
<220>
   <221> SITE
   <222> (9)..(9)
   <223> Norleucine
<400> 11
   Xaa Asp Xaa Asp Xaa Asp Xaa Asp Xaa
   1 5
<210> 12.
   <211> 9
   <212> PRT
   <213> Artificial sequence
<220>
   <223> Synthetic Construct
<400> 12
   Phe Asp Phe Asp Phe Asp Phe Asp Phe
   1 5
<210> 13
   <211> 9
   <212> PRT
   <213> Artificial sequence
<220>
   <223> Synthetic Construct
<400> 13
   Ser Asp Ser Asp Ser Asp Ser Asp Ser
   1 5
<210> 14
   <211> 9
   <212> PRT
   <213> Artificial sequence
<220>
   <223> Synthetic Construct
<400> 14
   Asp Asp Asp Asp Asp Asp Asp Asp Asp
   1 5
<210> 15
   <211> 7
   <212> PRT
   <213> Artificial sequence
<220>
   <223> Synthetic Construct
<220>
   <221> SITE
   <222> (2)..(2).
   <223> Norleucine
<220>
   <221> SITE
   <222> (4) .. (4)
   <223> Norleucine
<220>
   <221> SITE
   <222> (6).. (6)
   <223> Norleucine
<400> 15
   Asp Xaa Asp Xaa Asp Xaa Asp
   1 5
<210> 16
   <211> 7
   <212> PRT
   <213> Artificial sequence
<220>
   <223> Synthetic Construct
<400> 16
   Asp Phe Asp Phe Asp Phe Asp
   1 5
<210> 17
   <211> 7
   <212> PRT
   <213> Artificial sequence
<220>
   <223> Synthetic Construct
<400> 17
   Asp Ser Asp Ser Asp Ser Asp
   1 5
<210> 18
   <211> 7
   <212> PRT
   <213> Artificial sequence
<220>
   <223> Synthetic Construct
<400> 18
   Asp Asp Asp Asp Asp Asp Asp
   1 5
<210> 19
   <211> 11
   <212> PRT
   <213> Artificial sequence
<220>
   <223> Synthetic Construct
<400> 19
   Phe Gly Phe Ser Phe Ala Phe Asp Phe Gly Phe
   1 5 10
<210> 20
   <211> 13 <212> PRT
   <213> Artificial sequence
<220>
   <223> Synthetic Construct
<400> 20
   Phe Gly Phe Gly Phe Ser Phe Ala Phe Asp Phe Gly Phe
   1 5 10
<210> 21
   <211> 15
   <212> PRT
   <213> Artificial sequence
<220>
   <223> Synthetic Construct
<400> 21
   Phe Gly Phe Gly Phe Gly Phe Ser Phe Ala Phe Asp Phe Gly Phe
   1 5 10 15
<210> 22
   <211> 17
   <212> PRT
   <213> Artificial sequence
<220>
   <223> Synthetic Construct
<400> 22
   Phe Gly Phe Gly Phe Gly Phe Gly Phe Ser Phe Ala Phe Asp Phe Gly
   1 5 10 15
   Phe
<210> 23
   <211> 19
   <212> PRT
   <213> Artificial sequence
<220>
   <223> Synthetic Construct
<400> 23
   Phe Gly Phe Gly Phe Gly Phe Gly Phe Gly Phe Ser Phe Ala Phe Asp
   1 5 10 15
   Phe Gly Phe
<210> 24
   <211> 7
   <212> PRT
   <213> Artificial sequence
<220>
   <223> Synthetic Construct
<400> 24
   Gly Ser Phe Ala Phe Asp Phe
   1 5
<210> 25
   <211> 7
   <212> PRT
   <213> Artificial sequence
<220>
   <223> Synthetic Construct
<400> 25
   Gly Ser Gly Ala Phe Asp Phe
   1 5
<210> 26
   <211> 13
   <212> PRT
   <213> Artificial sequence
<220>
   <223> Synthetic Construct
<400> 26
   Phe Gly Phe Lys Phe Gly Phe Asp Phe Gly Phe Ala Phe
   1 5 10
<210> 27
   <211> 13
   <212> PRT
   <213> Artificial sequence
<220>
   <223> Synthetic Construct
<400> 27
   Phe Gly Phe Lys Phe Gly Phe Asp Phe Gly Phe Ser Phe
   1 5 10
<210> 28
   <211> 13
   <212> PRT
   <213> Artificial sequence
<220>
   <223> Synthetic Construct
<400> 28
   Phe Gly Phe Lys Phe Gly Phe Asp Phe Gly Phe Cys Phe
   1 5 10
<210> 29
   <211> 13
   <212> PRT
   <213> Artificial sequence
<220>
   <223> Synthetic Construct
<220>
   <221> SITE
   <222> (12).. (12)
   <223> nitropyridylthio activated cysteiyl
<400> 29
   Phe Gly Phe Lys Phe Gly Phe Asp Phe Gly Phe Xaa Phe
   1 5 10
<210> 30
   <211> 19
   <212> PRT
   <213> Artificial sequence
<220>
   <223> Synthetic Construct
<400> 30
   Phe Gly Phe Gly Phe Gly Phe Gly Phe Gly Phe Ser Phe Ala Phe Asp
   1 5 10 15
   Phe Gly Phe
<210> 31 <211> 23
   <212> PRT
   <213> Artificial sequence
<220>
   <223> synthetic Construct
<220>
   <221> SITE
   <222> (18)....(18)
   <223> diaminobutyryl residue
<220>
   <221> SITE
   <222> (20)..(20)
   <223> diaminobutyryl residue
<400> 31
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 32
   <211> 23
   <212> PRT
   <213> Artificial sequence
<220>
   <223> Synthetic Construct
<220>
   <221> SITE
   <222> (18)..(18)
   <223> diaminobutyryl residue
<220>
   <221> SITE
   <222> (20)..(20)
   <223> homoseryl residue
<400> 32
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 33
   <211> 23
   <212> PRT
   <213> Artificial sequence
<220>
   <223> Synthetic Construct
<220>
   <221> SITE
   <222> (18)..(18).
   <223> diaminobutyryl residue
<220>
   <221> SITE
   <222> (20)..(20)
   <223> aminobutyryl residue
<400> 33
   Phe Gly Phe Gly Phe Gly Phe Gly Phe Gly Phe Gly Phe Gly Phe Cys
   1               5                   10                  15
   Phe Xaa Phe Xaa Phe Gly Phe
               20
<210> 34
   <211> 23
   <212> PRT
   <213> Artificial sequence
<220>
   <223> Synthetic Construct
<220>
   <221> SITE
   <222> (18).. (18)
   <223> diaminobutyryl residue
<400> 34
   Phe Gly Phe Gly Phe Gly Phe Gly Phe Gly Phe Gly Phe Gly Phe Cys
   1               5                   10                  15
   Phe Xaa Phe Asp Phe Gly Phe
               20
<210> 35
   <211> 23
   <212> PRT
   <213> Artificial sequence
<220>
   <223> Synthetic Construct
<220>
   <221> SITE
   <222> (18)..(18)
   <223> homoseryl residue
<220>
   <221> SITE
   <222> (20)..(20)
   <223> diaminobutyryl residue
<400> 35
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 36
   <211> 23
   <212> PRT
   <213> Artificial sequence
<220>
   <223> Synthetic Construct
<220>
   <221> SITE
   <222> (18)..(18)
   <223> homoseryl residue
<220>
   <221> SITE
   <222> (20)..(20)
   <223> homoseryl residue
<400> 36
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 37
   <211> 23
   <212> PRT
   <213> Artificial sequence
<220>
   <223> Synthetic Construct
<220>
   <221> SITE <222> (18)..(18)
   <223> homoseryl residue
<220>
   <221> SITE
   <222> (20)..(20)
   <223> aminotiutyryl residue
<400> 37
   Phe Gly Phe Gly Phe Gly Phe Gly Phe Gly Phe Gly Phe Gly Phe Cys
   1 5 10 15
   Phe Xaa Phe Xaa Phe Gly Phe
   20
<210> 38
   <211>. 23
   C212> PRT
   <213> Artificial sequence
<220>
   <223> Synthetic Construct
<220>
   <221> SITE
   <222> (18).. (18)
   <223> homoseryl residue
<400> 38
<210> 39
   <211> 23
   <212> PRT
   <213> Artificial sequence
<220>
   <223> Synthetic Construct
<220>
   <221> SITE
   <222> (18)..(18)
   <223> aminobutyryl residue
<220>
   <221> SITE
   <222> (20)..(20)
   <223> diaminobutyryl residue
<400> 39
   Phe Gly Phe Gly Phe Gly Phe Gly Phe Gly Phe Gly Phe Gly Phe Cys
   1               5                   10                  15
   Phe Xaa Phe Xaa Phe Gly Phe
               20
<210> 40
   <211> 23
   <212> PRT
   <213> Artificial sequence
<220>
   <223> Synthetic Construct
<220>
   <221> SITE
   <222> (18)..(18)
   <223> Aminobutyryl residue
<220>
   <221> SITE
   <222> (20)..(20)
   <223> homoseryl residue
<400> 40
   Phe Gly Phe Gly Phe Gly Phe Gly Phe Gly Phe Gly Phe Gly Phe Cys
   1               5                   10                  15
   Phe Xaa Phe Xaa Phe Gly Phe
               20
<210> 41
   <211> 23
   <212> PRT
   <213> Artificial sequence
<220>
   <223> Synthetic Construct
<220>
   <221> SITE
   <222> (18)..(18)
   <223> aminobutyryl residue
<220>
   <221> SITE
   <222>. (20)..(20)
   <223> aminobutyryl residue
<400> 41
   Phe Gly Phe Gly Phe Gly Phe Gly Phe Gly Phe Gly Phe Gly Phe Cys
   1               5                   10                  15
   Phe Xaa Phe Xaa Phe Gly Phe
               20
<210> 42
   <211> 23
   <212> PRT
   <213> Artificial sequence
<220>
   <223> Synthetic Construct.
<220>
   <221> SITE
   <222> (18)..(18)
   <223> aminobutyryl residue
<400> 42
   Phe Gly Phe Gly Phe Gly Phe Gly Phe Gly Phe Gly Phe Gly Phe Cys
   1               5                   10                  15
   Phe Xaa Phe Asp Phe Gly Phe
               20
<210> 43
   <211> 23
   <212> PRT
   <213> Artificial sequence,
<220>
   <223> Synthetic Construct
<220>
   <221> SITE
   <222> (20)..(20)
   <223> diaminobutyryl residue
<400> 43
   Phe Gly Phe Gly Phe Gly Phe Gly Phe Gly Phe Gly Phe Gly Phe Cys
   1 5 10 15
   Phe Asp Phe Xaa Phe Gly Phe
   20
<210> 44
   <211> 23
   <212> PRT
   <213> Artificial sequence
<220>
   <223> Synthetic Construct.
<220>
   <221> SITE
   <222> (20)..(20)
   <223> homoseryl residue
<400> 44
   Phe Gly Phe Gly Phe Gly Phe Gly Phe Gly Phe Gly Phe Gly Phe Cys
   1 5 10 15
   Phe Asp Phe Xaa Phe Gly Phe
   20
<210> 45
   <211> 23
   <212> PRT
   <213> Artificial sequence
<220>
   <223> Synthetic Construct
<220>
   <221>. SITE
   <222> (20)..(20)
   <223> aminobutyryl residue
<400> 45
   Phe Gly Phe Gly Phe Gly Phe Gly Phe Gly Phe Gly Phe Gly Phe Cys
   1               5                   10                  15
   Phe Asp Phe Xaa Phe Gly Phe
               20
<210> 46
   <211> 23
   <212> PRT
   <213> Artificial sequence
<220>
   <223> Synthetic Construct.
<400> 46
   Phe Gly Phe Gly Phe Gly Phe Gly Phe Gly Phe Gly Phe Gly Phe Cys
   1               5                   10                  15
   Phe Asp Phe Asp Phe Gly Phe
               20
<210> 47
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl residue
<220>
   <221> SITE
   <222> (4)..(4)
   <223> diaminobutyryl residue
<400> 47
   Xaa Xaa Phe Xaa Phe Gly Phe
   1 5
<210> 48
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl residue
<220>
   <221> SITE
   <222> (4)..(4)
   <223> homoseryl residue
<400> 48
   Xaa Xaa Phe Xaa Phe Gly Phe
   1 5
<210> 49
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl desidue
<220>
   <221> SITE
   <222> (4).. (4)
   <223> aminobutyryl desidue
<400> 49
   Xaa Xaa Phe Xaa Phe Gly Phe
   1 5
<210> 50
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl residue
<400> 50
   Xaa Xaa Phe Asp Phe Gly Phe
   1 5
<210> 51
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <2210 SITE
   <222> (2)..(2)
   <223> homoseryl residue
<220>
   <221> SITE
   <222> (4).. (4)
   <223> diaminobutyryl residue
<400> 51
   Xaa Xaa Phe Xaa Phe Gly Phe
   1 5
<210> 52 <211> 7
   <212> PRT
   <213> Artificial sequence
<220>
   <223> Synthetic Construct
<220>
   <221> SITE
   <222> (1).. (1)
   <223> nitropyridylthio activated cysteiyl
<220>
   <221> SITE
   <222> (2).. (2)
   <223> homoseryl residue
<220>
   <221> SITE
   <222> (4).. (4)
   <223> homoseryl residue
<400> 52
   Xaa Xaa Phe Xaa Phe Gly Phe
   1 5
<210> 53
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2).. (2)
   <223> homoseryl residue
<220>
   <221> SITE
   <222> (4)..(4)
   <223> aminobutyryl residue
<400> 53
   Xaa Xaa Phe Xaa Phe Gly Phe
   1 5
<210> 54
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> homoseryl residue
<400> 54
   Xaa Xaa Phe Asp Phe Gly Phe
   1 5
<210> 55
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2).. (2)
   <223> aminobutyryl residue
<220>
   <221> SITE
   <222> (4).. (4)
   <223> diaminobutyryl residue
<400> 55
   Xaa Xaa Phe Xaa Phe Gly Phe
   1 5
<210> 56
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2).. (2)
   <223> aminobutyryl residue
<220>.
   <221> SITE
   <222> (4)..(4)
   <223> homoseryl residue
<400> 56
   Xaa Xaa Phe Xaa Phe Gly Phe
   1 5
<210> 57
   <211> 7
   <212> PRT
   <213> Artificial sequence
<220>
   <223> Synthetic Construct
<220>
   <221> SITE
   <222> (1).. (1)
   <223> nitropyridylthio activated cysteiyl
<220>.
   <221> SITE
   <222> (2)..(2)
   <223> aminobutyryl residue
<220>
   <221> SITE
   <222> (4)..(4)
   <223> aminobutyryl residue
<400> 57
   Xaa Xaa Phe Xaa Phe Gly Phe
   1 5
<210> 58
   <211> 7
   <212> PRT
   <213> Artificial -sequence
<220>
   <223> Synthetic Construct
<220>
   <221> SITE
   <222> (1)..(1)
   <223> nitropyridylthio activated cysteiyl
<220>
   <221> SITE
   <222> (2).. (2)
   <223> aminobutyryl residue
<400> 58
   Xaa Xaa Phe Asp Phe Gly Phe
   1 5
<210> 59
   <211> 7
   <212> PRT
   <213> Artificial sequence.
<220>
   <223> Synthetic Construct
<220>
   <221> SITE
   <222> (1)..(1)
   <223> nitropyridylthio activated cysteiyl
<220>
   <221> SITE
   <222> (4)..(4)
   <223> diaminobutyryl residue
<400> 59
   Xaa Asp Phe Xaa Phe Gly Phe
   1 5
<210> 60
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (4) ....(4)
   <223> homoseryl residue
<400> 60
   Xaa Asp Phe Xaa Phe Gly Phe
   1 5
<210> 61
   <211> 7
   <212> PRT
   <213> Artificial sequence
<220>
   <223> Synthetic Construct
<220>
   <221> SITE
   <222> (1).. (1)
   <223> nitropyridylthio activated cysteiyl
<220>
   <221> SITE
   <222> (4)..(4)
   <223> aminobutyryl residue
<400> 61
<21'0> 62
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<400> 62
<210> 63
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
   <222> (2)..(2)
   <223> diaminobutyryl residue
<220>
   <221> SITE
   <222> (4)..(4)
   <223> diaminobutyryl residue
<400> 63
   Xaa Xaa Phe Xaa Phe Gly Phe
   1 5
<210> 64
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<220>
   <221> SITE
<222> (2) .. (2)
   <223> homoseryl residue
<220>
   <221> SITE
   <222> (4)..(4)
   <223> homoseryl residue
<400> 64
   Xaa Xaa Phe Xaa Phe Gly Phe
   1 5
<210> 65
   <211> 7
   <212> PRT
   <213> Artificial sequence
<220>
   <223> Synthetic Construct
<220>
   <221> SITE
   <222> (1)...(1)
   <223> nitropyridylthio activated cysteiyl
<220>
   <221> SITE
   <222> (2).. (2)
   <223> aminobutyryl residue
<220>
   <221> SITE
   <222> (4).. (4)
   <223> aminobutyryl residue
<400> 65
   Xaa Xaa Phe Xaa Phe Gly Phe
   1 5
<210> 66
   <211> 7
   <212> PRT
   <213> Artificial sequence
<220>
   <223> Synthetic Construct
<220>
   <221> SITE
   <222> (1)..(1)
   <223> nitropyridylthio activated cysteiyl
<400> 66
   Xaa Asp Phe Asp Phe Gly Phe
   1               5

## Description

### TECHNICAL FIELD

The currant invention relates to novel methods of fabricating a dimeric capture agent at a surface. Also described are capture agents produced by the novel method, and arrays of such capture agents.

### BACKGROUND ART

The combinatorial production of molecules such as peptides is well known. This technique has provided a powerful tool, enabling the production of large libraries of molecules which can be used in techniques such as high throughout screening. Examples of' such techniques and their uses in identifying molecules of interest are disclosed in, for example, J. Org. Chem.,63, 8696, (1998), where a 1,000-peptide library of 3-mers was reacted with a dansyl 'tweezer' molecule in water. 3% of the library bound to the 'tweezer'. In J. Comb. Chem., 5, 794, (2003), a tripodal cyclotriveratrylene scaffold was described for split and mix synthesis and a library with ∼2,000 members was described.

Nature Biotechnology, 22, 568, (2004), Dario Neri et al, describes two libraries of organic molecule binders with attached DNA tags. DNA complementarity was used to make a higher diversity library. A further library was also made using triplex formation. 24 mer oligos were used for complex assembly. Organic molecule deconvolution was achieved by sequencing the attached DNA tags or by binding to oligonucleotide arrays. Binders were generated to protein with nM affinities.

Bioconjugate Chem., 12, 346, (2001), describes fabrication of peptide microarrays and small molecule microarrays. This document also discloses that chemoselective ligation can be used with peptides and slide surfaces. In this technique, an N-terminal cysteiyl residue reacts with an alpha keto aldehyde on the slide surface to give a thiazolidine ring. Others have used the free radical Michael addition between a free thiol and a maleimide.

Further, Chem. Commun., 581, (2005), describes a strategy to build complex libraries of cyclic peptides on a surface through photolithographic synthesis. A differential protection strategy is used for the combinatorial addition of side chains to a prefabricated core. US 5,491,074 discloses the association of peptide librairies.

In order to achieve the necessary diversity required for a meaningful library, the known methods require a large number of syntheses to be undertaken, thus increasing the time required, and cost of producing the library.

### DISCLOSURE OF INVENTION

It is an object of the current invention to provide a faster and cheaper method of producing a library of molecule having the required diversity.

The present invention provides a method of fabricating a set of dimeric peptide capture agents each of said capture agents being suitable for binding a ligand and comprising first and second peptide monomer units each comprising a ligand binding moiety and said first and second peptide monomer units being covalently linked to form a dimer, said method comprising the steps of:
a) synthesising in a combinatorial manner from first and second sets of amino acids, wherein each amino acid set is different, first and second peptide monomer units, each peptide monomer unit having a different primary amino acid sequence, each said first peptide monomer units comprising a first reactive group and an attachment moiety comprising hydrophobic or covalent means for immobilisiing the dimeric capture agent on a substrate and each said second peptide monomer unit comprising a second reactive group; and
b) reacting said first peptide monomer units with said second peptide monomer units such that the first and second reactive groups react to form a covalent bond whereby a combinatorially-varied set of dimeric peptide capture agents is formed, wherein the combinatorial synthesis is carried out on a solid phase and prior to step (b) said first and second peptide monomer units are cleaved from the solid phase.

Preferably, the reactive groups are thiol groups. More preferably, the method comprises covalently linking the capture agents to the substrate. More preferably, the method comprises attaching capture agents to the substrate by native chemical ligation between thioester-derivatised capture agents and cysteine-derivatised surfaces. More preferably the method comprises attaching the capture agents to the substrate by native chemical ligation between capture agents with N-terminal cysteines and thioester-derivatised surfaces. Alternatively, the method comprises immobilising the capture agent on the substrate by hydrophobic interaction.

Preferably the method includes the further step (c) of immobilising the set of dimeric peptide capture agents on a substrate by means of the attachment moiety,
wherein step (b) is performed before, simultaneously with or subsequent to step (c).
Preferably, the first and second peptide monomer units each comprise between 2 and 50 amino acids. More preferably, said first and second peptides comprise 3 to 10 ligand binding amino acid residues.

Preferably, the said first and second peptides include amino acids for ligand binding selected from amino acids with side chains providing a positive charge for ligand binding, amino acids providing a hydroxyl group capable of acting as a hydrogen bond donor and/or acceptor for ligand binding, amino acids providing a hydrophobic moiety for ligand binding and amino acids providing a negative charge for ligand binding.

Preferably the attachment moiety is formed by each peptide comprising a plurality of hydrophobic amino acids. More preferably, the first peptide comprises a primary structure comprising alternating hydrophobic and non-hydrophobic amino acid residues.

Preferably the method comprises selecting the hydrophobic amino acids from the group consisting of leucine, isoleucine, norleucine, valine, norvaline, methionine, tyrosine, tryptophan and phenylalanine.

Preferably, the first peptide has the sequence set out in SEQ ID No. 1.

Preferably the second peptide has the sequence set out in SEQ ID No. 2.

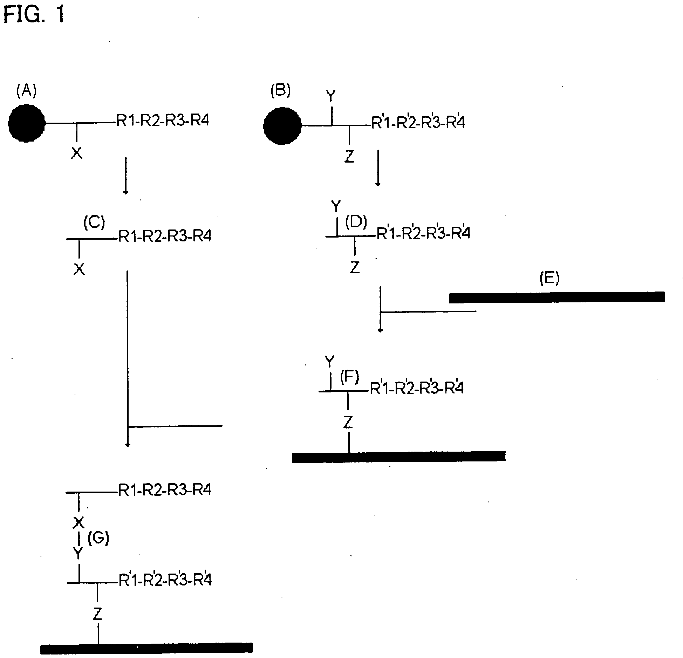

Schematic representations of possible methods of fabricating dimeric capture agents are shown in figures 1, 2 and 3 and described in Example 1.

There is also described herein a method of fabricating a dimeric capture agent for binding a ligand, or to form part of the set fabricated above said capture agent comprising at least first and second peptide monomers units,
said first monomer unit further comprising a first ligand-binding moiety, a first reactive group and an attachment moiety,
said second monomer unit further comprising a second ligand-binding moiety, and a second reactive group,
wherein the reactive groups may be the same or different for each monomer unit,
said method comprising the steps of;

### a) reacting the first monomer unit with the second monomer unit such that reactive groups present on the monomer units react to form a dimeric capture agent.

Preferably, the described method further includes the step of immobilising the capture agent on a substrate.

The present invention will now be further described. In the following passages, different embodiments' of the invention are defined in more detail. Each embodiment so defined may be combined with any other embodiment or embodiments unless clearly indicated to the contrary. In particular, any feature indicated as being preferred or advantageous may be combined with any other feature or features indicated as being preferred or advantageous.

The first and second peptide monomer units are covalently linked. It will be understood that immobilisation may be, for example, by covalent, or hydrophobic, interactions.

The attachment moiety comprises covalent or hydrophobic means for immobilising the capture agent on the substrate.

By polypeptide, it is meant a chain of 2 or more amino acids.

The first and second monomer units are synthesised. The first and second monomer units are synthesised on a solid phase and are different. The monomer units are cleaved from the solid phase prior to reacting said omits to form a co-valent bond.

Synthesis of polynucleotides and polypeptides is well known to those skilled in the art.

Syntheses of peptides and their salts and derivatives, including both solid phase and solution phase peptide syntheses, are well established. See, e.g., Stewart, et al. (1984) Solid Phase Peptide Synthesis (2nd Ed.); and Chan (2000) "FMOC Solid Phase Peptide Synthesis, A Practical Approach," Oxford University Press. Peptides may be synthesized using an automated peptide synthesizer (e.g., a Pioneer™ Peptide Synthesizer, Applied Biosystems, Foster City, CA). For example, a peptide may be prepared on Rink amide resin using FMOC solid phase peptide synthesis followed by trifluoroacetic acid (95%) deprotection and cleavage from the resin.

When the capture agent comprises a peptide dimes, the first and second peptides have different primary amino acid sequences.

It will be further apparent that the first and second peptides can be synthesised from first and second amino acid sets and that each amino acid set is different.

Preferably, the first and second peptides are between 2 and 100 amino acids in length, more preferably, 2 to 50 amino acids in length, and most preferably, 5 to 25 amino acids in length.

Preferably, each amino acid forming the ligand-binding moiety is selected from a set consisting essentially of fewer than 20 amino acids, more preferably fewer than 12 amino acids, even more preferably fewer than 6 amino acids and most preferably 4 amino acids.

It will be understood that each amino acid in the set can be an L-amino acid, a D-amino acid, an amino acid mimetic, a spacer amino acid, a beta amino acid, or any other chiral amino acid monomer. Preferably, the amino acids are L-amino acids and/or D-amino acids.

Preferably, each amino acid in the set is substantially enantiomerically pure.

Preferably, the first and second peptides for use in the method of the current invention each contains 10 or fewer ligand-binding residues; more preferably, 8 or fewer; more preferably, 6 or fewer; even more preferably, 4 or fewer; and most preferably 3 or fewer.

In a preferred embodiment, the reactive groups may be protected during peptide synthesis and deprotected prior to use in production of capture agents according to the first aspect. Such techniques are well known to those skilled in the art, for example, standard FMOC-based solid-phase peptide assembly. In this technique, resin bound peptides with protected side chains and free amino termini are generated. The amino groups at the N-terminus may then be reacted with any compatible carboxylic acid reactive group conjugate under standard peptide synthesis conditions. For example, cysteine with a trityl or methoxytrityl protected thiol group could be incorporated. Deprotection with trifluoroacetic acid would yield the unprotected peptide in solution.

Preferably, said reactive groups for use in any embodiment of the current invention are selected from, but not limited to, thiol groups, maleimide, cyclopentadiene, azide, phosphinothioesters, thioesters and (nitro) thiopyridine activated thiols. More preferably, the reactive groups are thiol groups. Preferably, when' the reactive groups are thiol groups, at least one thiol group is an activated thiol. Preferably, the thiol group is activated with either a thionitropyridyl or thiopyridyl group.

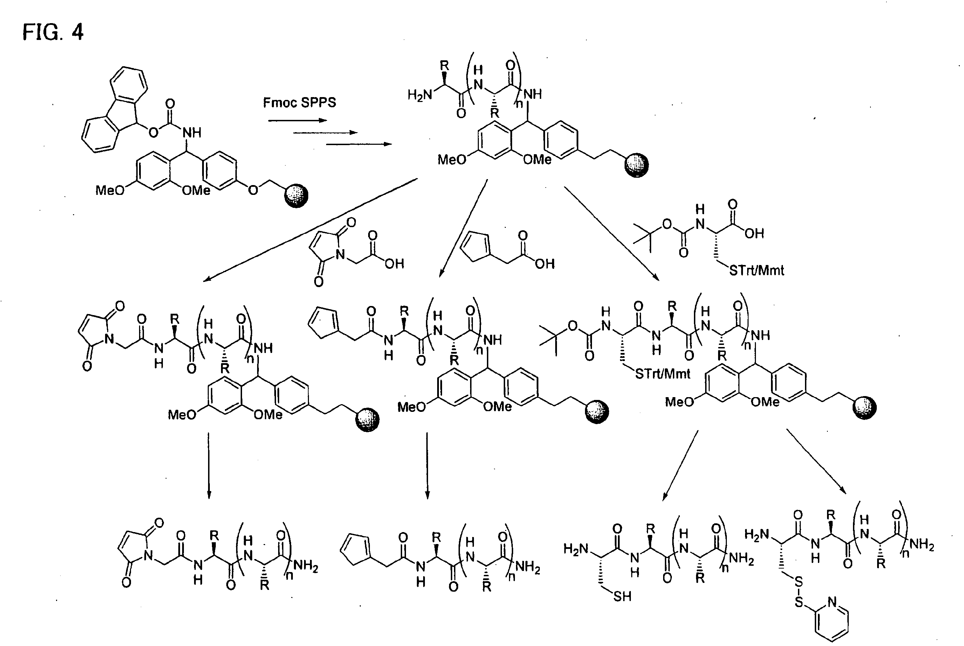

The reaction scheme outlined in Figure 4 shows a possible route for generating peptides comprising various reactive groups.

It will be understood that any suitable reaction may be used in the method of the current invention to form the multimeric capture agent, for example, Diels Alder reaction between e.g. cyclopentadienyl functionalised peptides and maleimide functionalised peptides, Michael reaction between a thiol functionalised peptide and a maleimide functionalised peptide, reaction between a thiol functionalised peptide and a peptide containing an activated thiol group (activated with, for example, a (nitro) thiopyridine moiety) to form a disulfide; Staudinger ligation between an azide functionalised peptide and a phosphinothioester functionalised peptide, and native chemical ligation between a thioester and a N-terminal cysteine.

It will be apparent that, depending upon the amino acid residues present in the peptides, the capture agents will have different characteristics. For example, the side chains may provide a positive charge for ligand binding. Preferably, the positive charge is provided by a lysyl residue (four CH₂ groups between the peptide chain and the positive charge), an ornithyl residue (three CH₂ groups between the peptide chain and the positive charge) or most preferably, a diaminobutyryl residue (with two CH₂ groups between the peptide chain and the positive charge).

The amino acid may alternatively provide a hydroxyl group capable of acting as a hydrogen bond donor and/or acceptor for ligand binding. Preferably, the hydroxyl group is provided by a seryl residue (one CH₂ group between the peptide chain and the OH group), or more preferably a homoseryl residue (with two CH₂ groups between the peptide chain and the OH group).

The amino acid may provide a hydrophobic moiety for ligand binding. Preferably, an alanyl residue (no CH₂ group between the peptide chain and the methyl group) or more preferably, an aminobutyryl residue (with one CH₂ group between the peptide chain and the methyl group) provides the hydrophobic moiety.

Alternatively, the amino acid may provide' a negative charge for ligand binding. Preferably, the negative charge is provided by a glutamyl residue (two CH₂ groups between the peptide chain and the carboxylate group), or more preferably, an aspartyl residue (one CH₂ group between the peptide chain and the carboxylate group).

The peptides are produced from the set of amino acids in a combinatorial manner, as is well known in the art, such that all possible combinations of amino acids present in the set may be produced, for example, if there are N amino acids in the set and the peptide is of length L, the complete set will comprise N^{L} peptides.

In a preferred embodiment, a subset of the possible combinatorial peptides which can be produced from any given set of amino acids will be employed. The subset can be determined through the inclusion of specific rules in the synthesis of the peptide, for example, maximum and minimum levels of each amino acid in the set can be provided, or maximum and minimum levels of the percentage hydrophobic amino acids incorporated can be provided.

The method of the current invention results in the production of capture agents having increased diversity. This arises from the fact that the combinatorially fabricated capture agents produced by the method are multimeric. For example, because the multimeric capture agent is a dimer, the possible diversity for any given length of ligand-binding moiety is squared due to the presence of two peptide chains. Therefore, it is necessary to carry out a reduced number of initial syntheses.

In a preferred embodiment, the peptides are immobilised such that the' side chains are located in space in a manner which is favourable for ligand-binding.

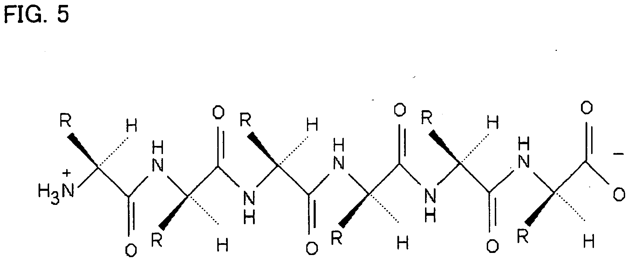

When the capture agents are immobilised through a covalent interaction, this may be achieved by, for example, synthesising peptides having alternating L- and D- amino acids as shown in Figure 5.

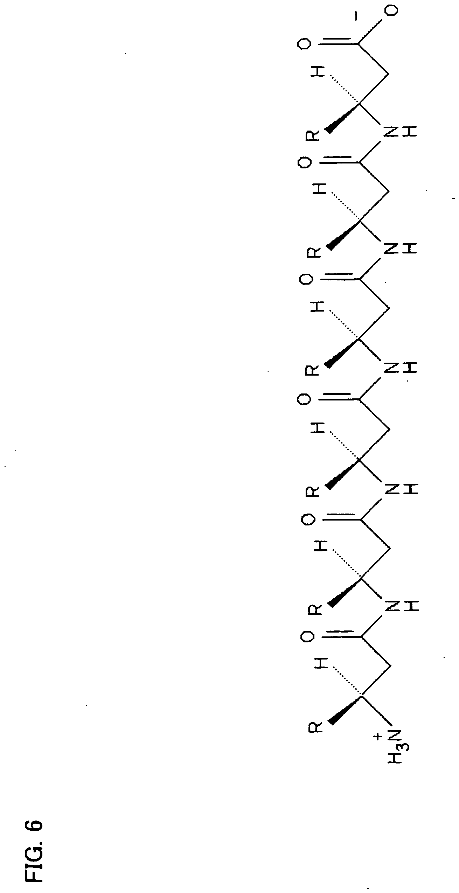

Alternatively, the peptides may be synthesised using a set comprising beta amino acids as shown in Figure 6, or any other chiral amino acid monomer with an even number of atoms per peptide repeat unit.

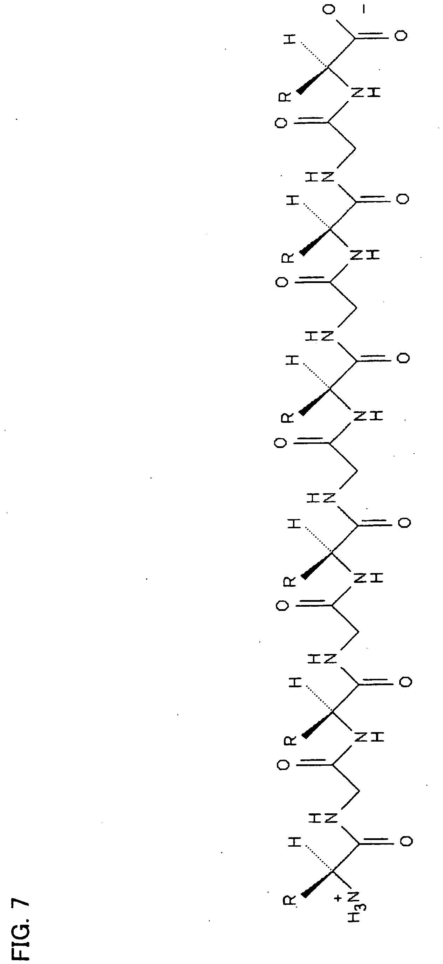

In one preferred embodiment, the peptides are synthesised such that only every second amino acid in the ligand binding region is varied, as shown in Figure 7.

This embodiment has the advantage that the side' chains are spaced in the most natural and advantageous manner for ligand-binding.

It will be obvious to the skilled person that each peptide can comprise one or more of the above types of amino acid and that the specific combinations employed will affect the characteristics of the capture agent containing the varying peptide chains.

When immobilisation of the capture agents to the substrate is via covalent attachment, the capture agents maybe "arrayed" on a substrate, and the array may take any convenient form. Thus, the method of the invention is applicable to all types of "high density" arrays, including single-molecule arrays.

Preferably, the covalently immobilised capture agents produced by the method of the first aspect are located at discrete spatially encoded loci on an array. Preferably, all of the said capture agents at a given locus on the array are comprised of the same pairs of peptides. More preferably, each locus on the array comprises a different capture agent.

When referring to immobilisation of molecules (e.g. peptides) to a substrate, the terms "immobilised" and "attached" are used interchangeably herein and in this embodiment, both terms are intended to encompass direct or indirect, covalent attachment, unless indicated otherwise, either explicitly or by context.

Certain embodiments of the invention may make use of substrates comprised of an inert substrate or matrix (e.g. glass slides, polymer beads etc) which has been "functionalised", for example by application of a layer or coating of an intermediate material comprising reactive groups which permit covalent attachment to biomolecules such as peptides. Examples of such supports include, but are not limited to, polyacrylamide hydrogels supported on an inert substrate such as glass. In such embodiments, the biomolecules (e.g. peptides) may be directly covalently attached to the intermediate material (e.g. the hydrogel) but the intermediate material may itself be non-covalently attached to the substrate or matrix (e.g. the glass substrate). The term "covalent attachment to a substrate" is to be interpreted accordingly as encompassing this type of arrangement.

In multi-peptide arrays, distinct regions on the array comprise multiple peptide molecules. Preferably, each site on the array comprises multiple copies of one individual peptide dimer.

Preferred reaction schemes for the covalent attachment of capture agents to substrates include, but are not limited to; reaction between sulfhydryls and maleimide derivatised surfaces, Diels-Alder reaction between maleimide derivatised surfaces and diene functionalised capture agents, azide and acetylene 3+2 cycloaddition, thiazolidine ring formation, and the modified Staudinger ligation. Alternatively, covalent attachment of the capture agents to the substrates may be effected in the reverse fashion, for example by reaction between a thiol derivatised surface and maleimide substituted peptides.

In a particularly preferred embodiment, native chemical ligation between thioester-derivatised capture agents and cysteine-derivatised surfaces that present both the amino group and the sulfhydryl group of the cysteine is used to covalently link the capture agents to the substrate.

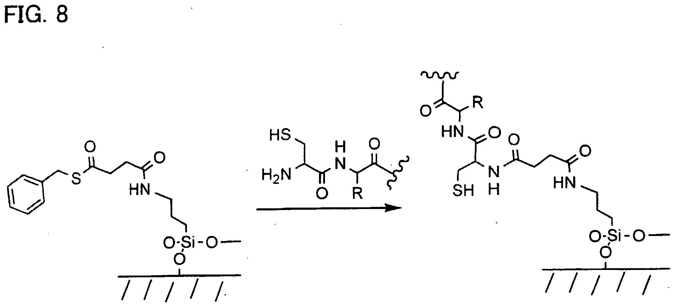

The most preferable reaction scheme uses native chemical ligation between capture agents with N-terminal cysteines and thioester-derivatised surfaces as shown in Figure 8.

Native chemical ligation generates a peptide bond between an N-terminal cysteine on a peptide and a surface-attached thioester. A particular advantage of this embodiment of the current invention is that protection of peptide side chains is not required. A further particular advantage of this embodiment of the current invention is that the resulting surface-attached peptide has an internal cysteine that may be exploited for the formation of dimeric receptors by disulfide bond formation, or reaction between a thiol and a maleimide functionalised peptide.

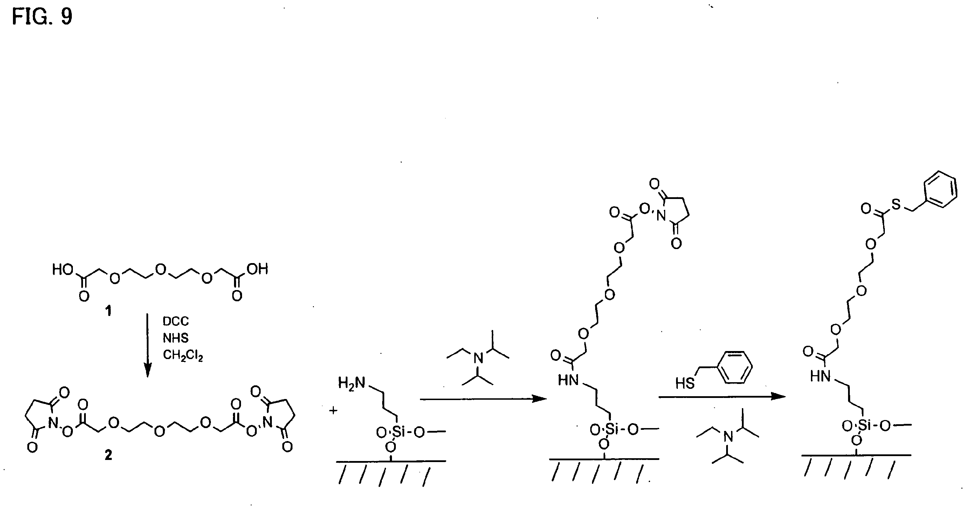

The preferred reaction scheme for the preparation of thioester functionalised glass surfaces is shown in Figure 9.

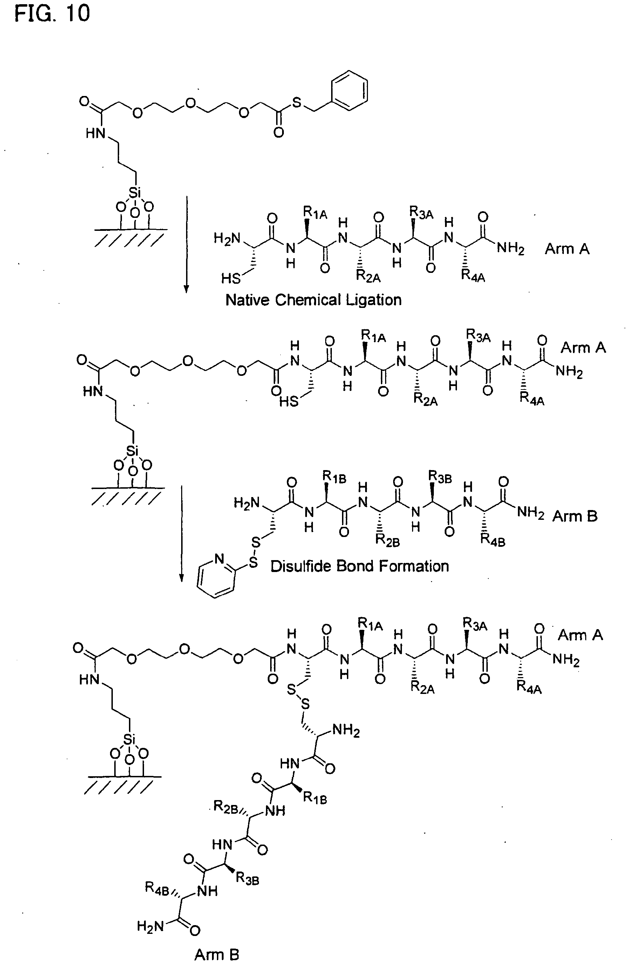

If the capture agent is assembled on the substrate surface, a preferred reaction scheme is shown in Figure 10. In this embodiment, the first peptide is covalently bound to the functionalised substrate via reaction between a thioester group and an N-terminal cysteine residue via native chemical ligation. The multimeric capture agent is produced by formation of disulfide bonds between the peptides.

A more preferable route for the preparation of thioester surfaces involves the reaction between an aminated surface and thiolane 2,4-diones of the type shown below:

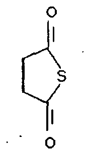

Thioester surfaces may also be made by derivatising hydroxylated surfaces with thioester silylchloride conjugates of the type shown below.

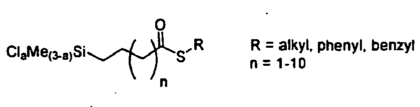

When the capture agent is immobilised to a substrate through a covalent bond, the dimer may be constructed wherein the first and second peptides each have a thiol group (which may or may not be activated) located at a site in the peptide sequence that is on the N-terminal side of the ligand-binding site or on the C-terminal side of the ligand-binding site or located internally within a bipartite ligand-binding site. In any of these embodiments, it will be clear that the thiol group moieties may have the same or opposite orientation as the ligand-binding residues and that the location of each thiol group in the first and second peptides is independent.

In an alternative embodiment, the capture agent is immobilised on the substrate by a hydrophobic interaction between the capture agent and the substrate.

In this embodiment, the capture agents fabricated according to the method of the current invention, comprise at least two peptide monomer units, the first and second monomer units each comprising at least one hydrophobic moiety, at least one reactive group and at least one ligand-binding moiety.

The capture agent fabricated according to the method comprises two peptides, the first and second peptides each comprising at least one hydrophobic amino acid residue, at least one reactive group, and at least one ligand-binding moiety, wherein, the at least one hydrophobic amino acid residue and the at least one ligand-binding moiety are positioned in the peptide primary structure so as to result in a hydrophobic face, and a substantially non hydrophobic ligand-binding face.

It will be understood that in this' embodiment of the method of the current invention, the hydrophobic face of the peptides forms the attachment moiety.

Preferably, each peptide comprises a plurality of hydrophobic amino acids forming the attachment moiety.

Preferably, the .first peptide for use in the method of the current invention comprises 4 to 40 hydrophobic amino acid residues, more preferably 6 to 25 and most preferably 6 to 12.

Preferably, the ligand-binding moiety comprises at least one amino acid. More preferably, the ligand-binding moiety comprises a plurality of amino acids.

It will be understood that amino acids positioned on the ligand-binding face may also include hydrophobic residues, for example, aminobutyrate residues.

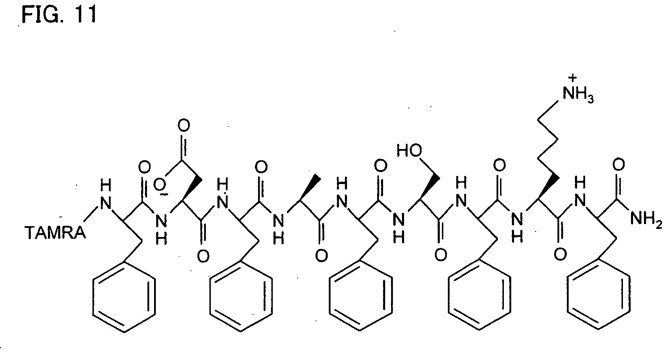

Preferably, each peptide for use in this embodiment comprises a primary structure comprising alternating hydrophobic and non hydrophobic amino acid residues, as shown in Figure 11.

It will be understood by the skilled person that other peptide sequences which result in distribution of the side chains so as to result in a hydrophobic and substantially non hydrophobic face can be easily designed, for example, there may be three non hydrophobic amino acid residues between hydrophobic residues, or any combination of odd numbers of amino acids. Alternatively, the' peptide may comprise a combination of, for example, L-, D-, and beta- amino acids so as to result in a hydrophobic and a substantially non hydrophobic face.

Preferably, each amino acid positioned so as to be located on the ligand-binding face is selected from a set consisting essentially of fewer than 20 amino acids, more preferably fewer than 12 amino acids, even more preferably fewer than 6 amino acids and most preferably 4 amino acids.

Preferably, each peptide for use in this embodiment comprises 10% to 90% hydrophobic amino acid residues, more preferably, 20% to 80%, even more preferably, 30% to 70%, and most preferably 40% to 60% hydrophobic amino acid residues.

In a particularly preferred embodiment, the first peptide comprises 50% hydrophobic amino acid residues.

Preferably, the hydrophobic amino acids are selected from the group consisting of leucine, isoleucine, norleucine, valine, norvaline, methionine, tyrosine, tryptophan and phenylalanine. More preferably, the hydrophobic amino acids are phenylalanine.

In a preferred embodiment, the capture agent is located on a hydrophobic substrate such that the substantially non hydrophobic ligand-binding face is accessible for ligand-binding.

It will be understood that the substrate for use in the method may be any suitable hydrophobic substrate, for example, gold modified by hydrophobic organic thiol treatment, glass modified by surface treatment, or plastic. Preferably, the substrate is plastic.

Alternatively, the substrate may be coated in a hydrophobic compound which allows the capture agents to be immobilised thereon in the presence of a substantially aqueous solvent.

In a preferred embodiment, the second peptide for use in this embodiment of the current invention comprises fewer amino acids than the first peptide, and contains fewer hydrophobic residues such that the interaction between the peptide and the hydrophobic surface is relatively weak.

It will be apparent to the skilled person that the length of the first and second peptides and the numbers of hydrophobic amino acid residues required to retain them on the substrate will depend upon the hydrophobicity of the surface and on the hydrophobic amino acids present in the first and second peptides, and also on the nature of the ligand to be bound.

It will also be readily apparent to the skilled person that the amount of peptide retained at the substrate will depend upon the stringency of washing to which the substrate is subjected. Preferably, after immobilisation of the peptides, the substrate is washed with, for example, 1.0 M NaCl in 10 mM tris-HCl (pH 8.0).

Preferably, the second peptide comprises 1-6 hydrophobic amino acid residues, more preferably, 2-5, and most preferably 2-4 hydrophobic amino acid residues on the hydrophobic face.

Preferably, the ligand-binding residues are positioned on the substantially non hydrophobic ligand-binding face.

When the capture agents are immobilised on the substrate by a hydrophobic interaction, the reactive groups may be located in the primary peptide structure of the first and second peptides at any suitable position, for example, the reactive groups may be positioned in the primary peptide sequence such that they are positioned on the substantially non hydrophobic ligand-binding face of the peptides and located on the N-terminal side of the ligand-binding site.

Alternatively, the reactive groups may be located in the primary peptide structure of the first and second peptides such that they are positioned on the substantially non hydrophobic ligand-binding face of the peptides, and in the first peptide, on the N-terminal side of the ligand-binding site, and in the second peptide to the C-terminal side of the ligand-binding site.

In a further embodiment, the reactive group may be located in the primary peptide structure of the first and second peptides such that in the first peptide, it is positioned on the substantially non hydrophobic ligand-binding face of the peptides and to the N-terminal side of the ligand-binding site, and in the second peptide it is located on the opposite (hydrophobic) face to the ligand-binding site and to the C-terminal side of the ligand-binding site.

In a preferred embodiment, the reactive group on the first peptide is located in the primary amino acid structure on the substantially non hydrophobic ligand-binding face and to the N-terminal side of the ligand-binding site and in the second peptide, in the hydrophobic face and to the N-terminal side of the ligand-binding site as shown in Figure 10.

Preferably in this embodiment, the capture agents are bound to the substrate so as to produce an array. It will be understood that the array may take any' convenient form. Thus, the method of the invention is applicable to all types of "high density" arrays, including single-molecule arrays.

Preferably, the array comprises a number of discrete addressable spatially encoded loci. Preferably, each locus on the array comprises a different capture agent, and more preferably each locus comprises multiple copies of the capture agent.

When referring to immobilisation of molecules (e.g. peptides) to a substrate, the terms "immobilised" and "attached" are used interchangeably herein and both terms are intended to encompass hydrophobic interactions, unless indicated otherwise, either explicitly or by context. Generally all that is required is that the molecules (e.g. peptides) remain immobilised or attached to the substrate under the conditions in which it is intended to use the substrate, for example in applications requiring peptide ligand-binding.

Certain embodiments of the invention may make use of solid supports comprised of an inert substrate or matrix (e.g. glass slides, polymer beads etc) which has been "functionalised", for example by application of a layer or coating of an intermediate material comprising reactive groups which permit hydrophobic attachment of biomolecules such as peptides.

In multi-peptide arrays, distinct regions on the array comprise multiple peptide molecules. Preferably, each site on the array comprises multiple copies of one individual peptide.

In a particularly preferred embodiment, the first peptide has the structure set out in SEQ ID No 1;
(Phe-Gly)ₙ-Phe-Cys-Phe-X-Phe-Y-Phe-Z-Phe-Gly-Phe
where X, Y, and Z are the ligand-binding residues and Cys provides a nucleophilic thiol used for dimer formation.

The second peptide has the preferred structure set out in SEQ ID No 2;
CysS(N)P-X'-Phe-Y'-Phe-Z'-Phe
where X', Y', and Z' are the ligand-binding residues and CysS(N)P is an activated thiol used for dimer formation (most preferably activated with either a thionitropyridyl group or a thiopyridyl group). ,

It is to be understood that the preceding preferred embodiments are by way of example only and are not to be taken to be limiting. It will be apparent to the skilled person that many other reactive groups and activating groups can be employed in the methods of the current invention.

In the most preferred embodiments, the capture agents fabricated according to the method of the current invention are dispensed onto a suitable substrate to form an addressable spatially encoded array of combinatorially varying dimers. Preferably, the peptides are individually dispensed on to the substrate using a non-contact dispenser (Piezorray System, Perkin Elmer LAS) and assembled in situ.

Also described herein is a capture agent fabricated according to the method described herein

Also described herein, is a substrate on which is immobilised at least one capture agent fabricated according to the method described herein

Preferably, the capture agent is immobilised through a covalent or hydrophobic interaction.

Also described herein is a kit comprising a multimeric capture agent fabricated according to the method described herein and a suitable substrate for immobilisation.

Alternatively, the kit may comprise first and second monomer units produced for use in the method described herein . Preferably, the kit further comprises a suitable substrate.

Also described herein is a method of identifying a multimeric capture agent fabricated according to the method which binds to a ligand of interest, said method comprising producing an array of combinatorial capture agents according to any previous aspect, contacting the ligand of interest with the array, and identifying to which capture agent(s) the ligand binds.

When referring to the binding of ligands to the immobilised peptides in any embodiment, the term bind is intended to encompass direct or indirect, covalent or non-covalent attachment, unless indicated otherwise, either explicitly or by context. In certain embodiments of the invention, covalent attachment may be preferred, but generally all that is required is that the ligands remain bound to the immobilised peptide under the conditions in which it is intended to use the substrate, for example in applications requiring further ligand receptor interactions.

It will be apparent to the skilled person that the binding of the ligand to a capture agent can be identified in various ways known in the art, for example, the ligand or the capture agent may be' labelled so that the location on the array to which the ligand binds can be identified. This label may, for example, be a radioactive or fluorescent label using, for example, fluorophores. Alternatively, binding of the ligand of interest to a capture agent may be detected by a variety of other techniques known in the art, for example, calorimetry, absorption spectroscopy, NMR methods, atomic force microscopy and scanning tunnelling microscopy, electrophoresis or chromatography, mass spectroscopy, capillary electrophoresis, surface plasmon resonance detection, surface acoustic wave sensing and numerous microcantilever-based approaches.

It will be understood that the dimeric capture agents arrays produced by the methods of the current invention can be used to identify any analyte of choice, since the specific ligand which will be bound by the capture agent will be dependent upon the length and sequence of the peptides from which the capture agent is formed. In preferred embodiments, the ligand comprises a eukaryotic cell, a prokaryotic cell, a virus, a bacteriophage, a prion, a spore, a pollen grain, an allergen, a nucleic acid, a protein, a peptide, a carbohydrate, a lipid, an organic compound, or an inorganic compound. The ligands are preferably physiological or pharmacological metabolites and most preferably physiological or pharmacological metabolites in human or animal bodily fluids that may be used as diagnostic or prognostic healthcare markers.

Additional objects, features, and strengths of the present invention will be made clear by the description below. Further, the advantages of the present invention will be evident from the following explanation in reference to the drawings.

### BRIEF DESCRIPTION OF DRAWINGS

The invention will be further understood with reference to the following experimental examples and accompanying figures in which:-
Figure 1 shows, a schematic representation of a first method of fabricating a capture agent
Figure 2 shows a schematic representation of a second method of fabricating a capture agent .
Figure 3 shows a schematic representation of a further method of fabricating a capture agent.
Figure 4 shows a possible route for generating peptides comprising various reactive groups.
Figure 5 shows a peptide comprising alternating L- and D-amino acids.
Figure 6 shows a peptide comprising beta amino acids.
Figure 7 shows a peptide wherein every second amino acid is varied.
Figure 8 shows schematically the method of native chemical ligation between capture agents with N-terminal and thioester-derivatised surfaces.
Figure 9 shows the preferred reaction scheme for the preparation of thioester functionalised glass.
Figure 10 shows a preferred reaction scheme for the fabrication of dimeric capture agents at a substrate surface.
Figure 11 shows a peptide comprising alternating hydrophobic and non hydrophobic amino acids.

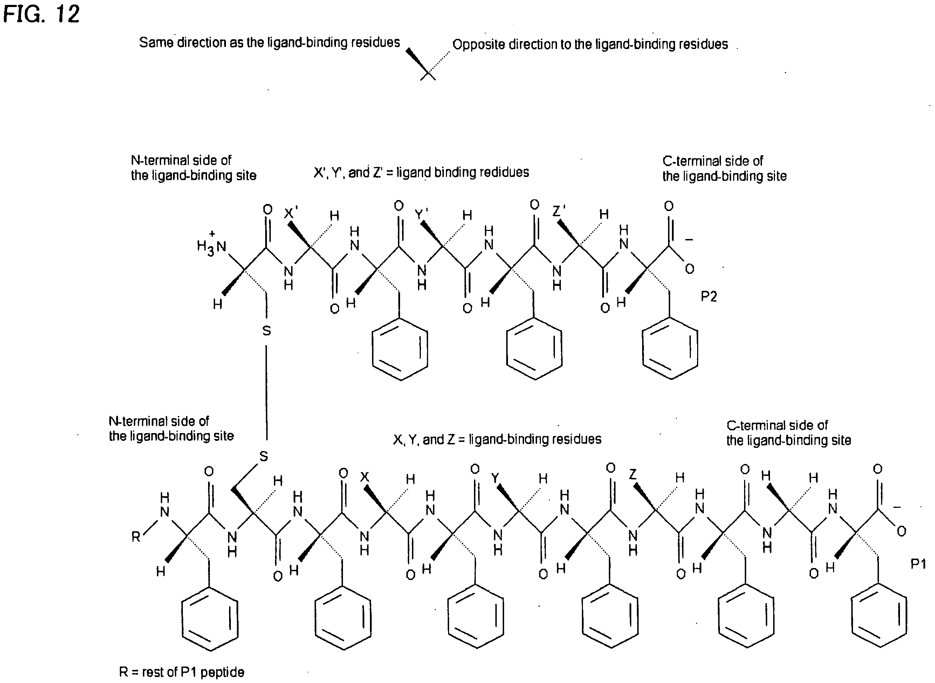

Figure 12 shows an example of a dimeric capture agent having a hydrophobic face and a substantially non-hydrophobic ligand-binding face.
Figure 13 is a graphical representation showing the locations of various hydrophobic capture agents in a 96 well plate.
Figure 14 shows fluorescence images of the 96 well plate of figure 11 indicating the presence of the various peptides in the wells.
Figure 15 shows a graphical representation of the quantified results of the 400V scan of Figure 14.
Figure 16A shows fluorescence images indicating the retention of polypeptides P1-1 to P1-5 and P2-1 to P2-2 on a polypropylene surface.
Figure 16B shows fluorescence images indicating the retention of polypeptides P1-1 to P1-5 and P2-1 to P2-2 on a polypropylene surface.
Figure 17 shows a graphical representation of the quantified results of Figure 16A,B.

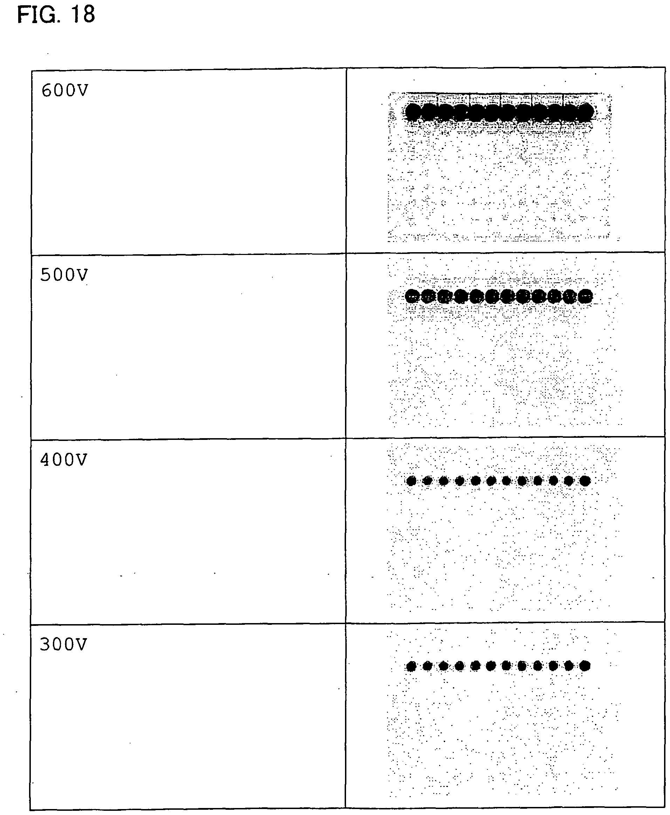

Figure 18 shows fluorescence images indicating the pH resistance of the peptide 2DOS-2 deposited on to a polypropylene hydrophobic surface.
Figure 19 shows a graphical representation of the quantified results of the 300V scan of Figure 18.
Figure 20 shows fluorescence images indicating the time dependent persistence of the peptide 2DOS-2 deposited on to a polypropylene hydrophobic surface in the presence of an aqueous buffer.
Figure 21 shows a graphical representation of the results of the 300V scan of Figure 20.
Figure 22 is a graphical representation showing the location of various hydrophobic peptides added to flat bottomed and V-bottomed polypropylene 96 well plates.
Figure 23 shows fluorescence images of the plates of Figure 22 showing retention of the hydrophobic peptides with and without washing.
Figure 24 is a graphical representation of the results of the 500V scan of Figure 23, for the V-bottomed plates.
Figure 25 is a graphical representation of the results of the 500V scan of Figure 23 for the flat bottomed plates:
Figure 26 is a graphical representation showing the location of various hydrophobic peptides added to polypropylene and polystyrene V-bottomed 96 well plates.
Figure 27 shows fluorescence images of the plates of Figure 26 showing retention of the hydrophobic peptides with and without washing.
Figure 28 is a graphical representation showing the percentage retention of the various peptides in the polypropylene and polystyrene plates of Figure 26 after washing.
Figure 29A shows fluorescence images of the microtitre plate from the experiment using the 'liquid phase' protocol.
Figure 29B is a graphical representation of the data from the fluorescence image shown in Table 21.
Figure 30A shows fluorescence images of the microtitre plate from the experiment using the 'co-drying' protocol.
Figure 30B is a graphical representation of the data from the fluorescence image shown in Table 22.
Figure 31 shows fluorescence images indicating the yield of dimer formation on polypropylene sheets.
Figure 32 shows a fluorescence images of a 256-element microarray of peptide dimers.

### BEST MODE FOR CARRYING OUT THE INVENTION

As used herein, the term spacer amino acid refers to an amino acid, a synthetic amino acid, an amino acid analogue or amino acid mimetic in which the side chains play no part in ligand-binding.

As used herein, the term capture agent refers to a dimeric molecule having a structure such that when a ligand is brought into contact with the capture agent it is bound thereto.

As used herein, the term peptide refers to a chain comprising two or more amino acid residues, synthetic amino acids, amino acid analogues or amino acid mimetics, or any combination thereof. The terms peptide, oligopeptide and polypeptide are used interchangeably in this specification.

As used herein, the term's oligonucleotide and polynucleotide refer to a chain of two or more nucleotides, and are used interchangeably in this specification.

As used herein, the term substantially enantiomerically pure indicates that the residue comprises substantially one type of isomer, with any other isomeric forms being there only as an impurity.

As used herein, the term located in space in a manner favourable to ligand-binding indicates that the side chains of the peptides which make up the multimeric capture agent are positioned such that they are able to contact and interact with a ligand.

As used herein, the term substantially non hydrophobic means comprising substantially more hydrophilic residues than hydrophobic residues.

### Example 1

Figure 1 shows a schematic representation of a method of producing capture' agents

A first set of monomer units (A) is prepared on a solid phase. The monomer units comprise a ligand-binding moiety (R1 - R4) and a reactive group X. If wished X may be protected during synthesis and then deprotected before use.

A second set of monomer units (B) is prepared on a solid phase. These monomer units comprise a ligand-binding moiety (R'1 - R'4), a reactive group Y, which may be protected during synthesis and then deprotected before use, and an attachment moiety Z. If wished Z may also be protected during synthesis and then deprotected before use.

Each of the monomer units in set (A) is cleaved off the solid support to give monomer units (C) in solution.

Each of the monomer units in set (B) is cleaved off the solid support to give monomer units (D) in solution.

Each of the monomer units in set D is then contacted with the surface of a solid support (E) at a spatially encoded location in an array such that Z is used to bring about attachment to the said surface.

Reactions are then performed wherein surface-bound monomer units (F) from set D are reacted with an excess or equimolar amount of a given solution phase monomer unit (C) such that residue X reacts with residue Y to form a dimeric structure (G) bound to the solid phase.

The arrayed and spatially encoded dimeric structures (G) can then be used for binding to ligands of interest that will bind with suitable affinity, and selectivity.

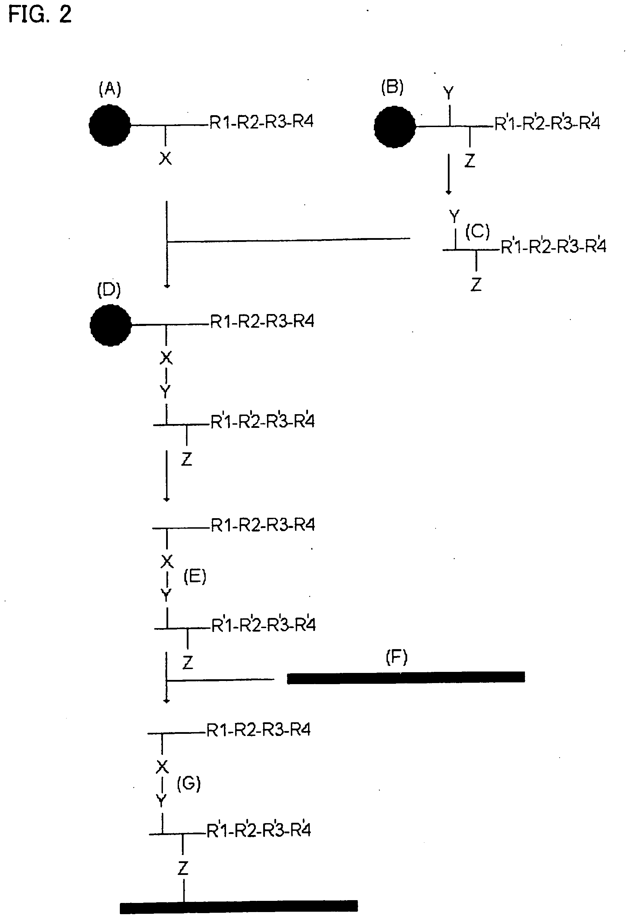

Figure 2 shows a schematic representation of a further method of producing capture agents.

In this embodiment, a first set of monomer units (A) is prepared on a solid phase. The monomer units comprise a ligand-binding moiety (R1 - R4) and a reactive group X, which may be protected during synthesis and then deprotected before use.

A second set of monomer units (B) is also prepared on a solid phase. These monomer units comprise a ligand-binding. moiety (R'1 - R'4) and a reactive group Y, which may be protected during synthesis and then deprotected before use, and an attachment moiety Z. If wished Z may also be protected during synthesis and then deprotected before use.

Each of the monomer units (B) is cleaved off the solid support to give monomer units (C) in solution. Reactions are then performed wherein a given solid phase-bound monomer unit from set (A) is reacted with an excess of a given solution phase monomer unit (C) such that residue X reacts with residue Y to form a dimeric structure (D) bound to the solid phase.

Each dimeric structure (D) bound to the solid phase is then cleaved off the solid support to give a solution phase dimeric structure (E).

Each solution phase dimeric structure (E) is finally contacted with a solid surface (F) at a spatially encoded location in an array such that group Z is used to attach the dimeric structure to the said surface.

The arrayed and spatially encoded dimeric structures (G) can then be used for binding to ligands of interest that will bind with suitable affinity, and selectivity.

The positions of X, Y, and Z in the figures are merely illustrative and should not be seen as limiting to the invention.

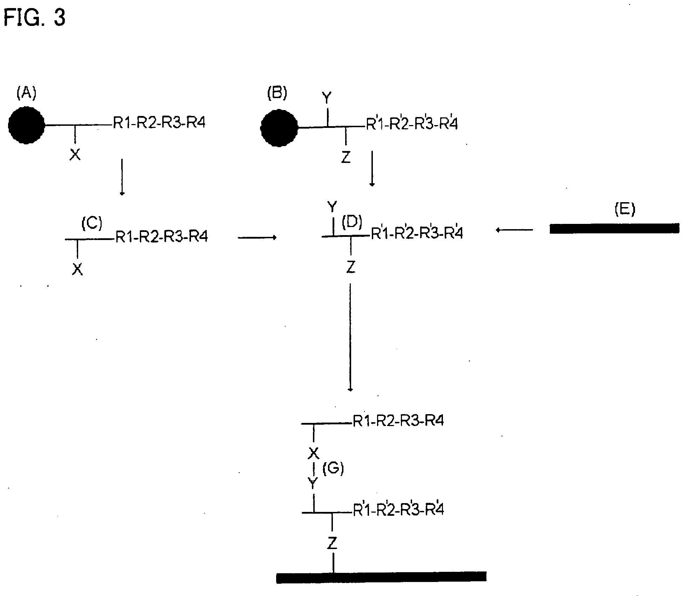

Figure 3 shows a schematic representation of a further method of producing capture agents.

A first set of monomer units (A) is prepared on a solid phase. The monomer units comprise a ligand-binding moiety (R1 - R4) and a reactive group X. If wished X may be protected during synthesis,and then deprotected before use.

A second set of monomer units (B) is prepared on a solid phase. These monomer units comprise a ligand-binding moiety (R'1 - R'4), a reactive group Y, which may be protected during synthesis and then deprotected before use, and an attachment moiety Z. If wished Z may also be protected during synthesis and then deprotected before use.

Each of the monomer units in set (A) is cleaved off the solid support to give monomer' units (C) in solution.

Each of the monomer units in set (B) is cleaved off the solid support to give monomer units (D) in solution.

Each of the monomer units in set D is then contacted with an excess or equimolar amount of a given solution phase monomer unit (C) and the surface of a solid support (E) at a spatially encoded location in an array such that Z is used to bring about attachment to the said surface and such that residue X reacts with residue Y to form a dimeric structure (G) bound to the solid phase.

The arrayed and spatially encoded dimeric structures (G) can then be used for binding to ligands of interest that will bind with suitable affinity, and selectivity.

The most significant advantage of the current invention is the 'squaring' (or raising to a higher power) of sequence diversity by the combinatorial joining of pairs (or greater numbers) of monomer units at the array surface.

### Example 2

The following series of peptides were synthesised in order to demonstrate peptide self-assembly into an organic solvent layer or onto a hydrophobic surface driven by entropic effects in an aqueous solvent in contact with the said organic solvent layer or hydrophobic surface.

All peptides are labelled with the rhodamine dye TAMRA at the N-terminus. A mixture of the 5-TAMRA and 6-TAMRA isomers was used for the labelling.

| 5-carboxytetramethylrhodamine (5-TAMRA) | 6-carboxytetramethylrhodamine (6-TAMRA) |
|---|---|
| 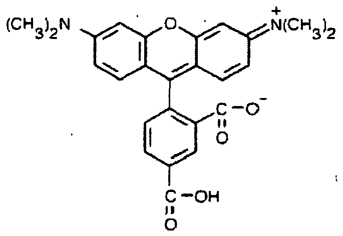 | 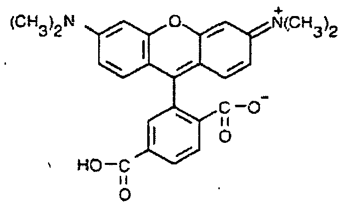 |
| Spectrum | Spectrum |
| 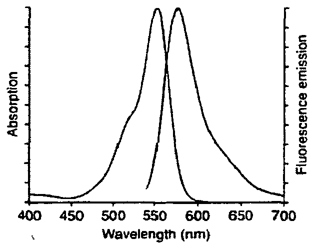 | 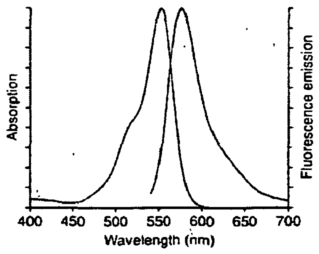 |

In the following, the residue side chains projecting in front of the plane of the paper represent the combinatorially varied 'ligand-binding face'. The residue side chains projecting behind the plane of the paper represent the 'hydrophobic face' (or negative control residues).

In the set of peptides 2DOS-1 to 2DOS-8, a mixture of four side chains (aspartyl, alanyl, seryl, and lysyl) has been used. In the set of peptides 2DOS-9 to 2DOS-16, four hydrophilic (aspartyl) chains have been used.

In the set of peptides 2DOS-1 to 2DOS-4 and the set of peptides 2DOS-9 to 2DOS-12, five residue side chains have been used for the 'hydrophobic face' (or negative control residues). In the set of peptides 2DOS-5 to 2DOS-8 and the set of peptides 2DOS-13 to 2DOS-16, three residue side chains have been used for the 'hydrophobic face' (or negative control residues).

For peptides 2DOS-1; 2DOS-5, 2DOS-9, and 2DOS-13, norleucyl residues have been used for the 'hydrophobic face'. For peptides 2DOS-2, 2DOS-6, 2DOS-10, and 2DOS-14, phenylalanyl residues have been used for the 'hydrophobic face'. For peptides 2DOS-3, 2DOS-7, 2DOS-11, and 2DOS-15, seryl residues have been used as a weak negative control for the 'hydrophobic face'. For peptides 2DOS-4, 2DOS-8, 2DOS-12, and 2DOS-16, aspartyl residues have been used as a strong negative control for the 'hydrophobic face':

**Table 1**

| Peptide name | Peptide sequence | Peptide structure |
|---|---|---|
| 2DOS-1 . | N-TAMRA-Norleu-A sp-Norleu-Ala-No rleu-Ser-Norleu-Lys-Norleu-C | 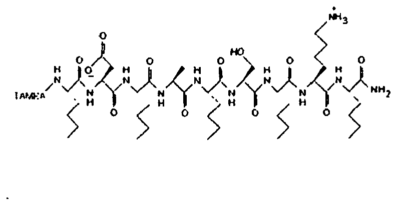 |
| 2DOS-2 | N-TAMRA-Phe-Asp-Phe-Ala-Phe-Ser-Phe-Lys-Phe-C | 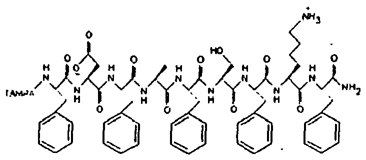 |
| 2DOS-3 | N-TAMRA-Ser-Asp-Ser-Ala-Ser-Ser-Ser-Lys-Ser-C | 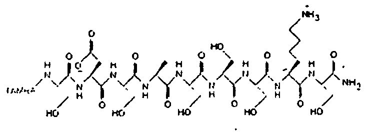 |
| 2DOS-4 | N-TAMRA-Asp-Asp-Asp-Ala-Asp-Ser-Asp-Lys-Asp-C | 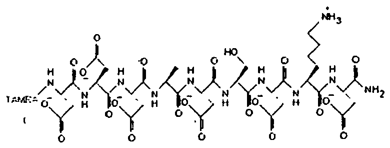 |
| 2DOS-5 | N-TAMRA-Asp-Norl eu-Ala-Norleu-Se r-Norleu-Lys-C | 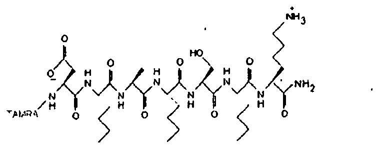 |
| 2DOS-6 | N-TAMRA-Asp-Phe-Ala-Phe-Ser-Phe-Lys-C | 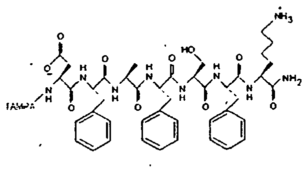 |
| 2DOS-7 | N-TAMRA-Asp-Ser-Ala-Se-r-Ser-Ser-Lys-C | 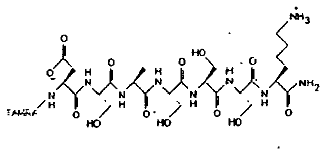 |
| 2DOS-8 | N-TAMRA-Asp-Asp-Ala-Asp-Ser-Asp-Lys-C | 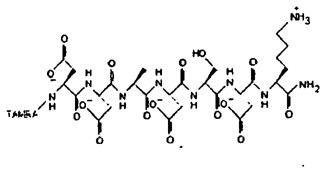 |
| 2DOS-9 | N-TAMRA-Norleu-A sp-Norleu-Asp-No rleu-Asp-Norleu-Asp-Norleu-C' | 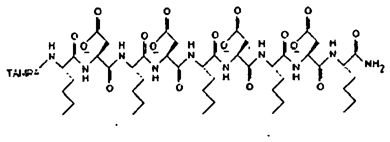 |
| 2DOS-10 | N-TAMRA-Phe-Asp-Phe-Asp-Phe-Asp-Phe-Asp-Phe-C | 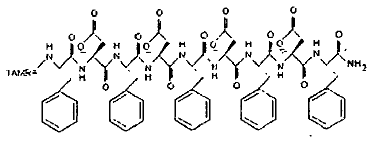 |
| 2DOS-11 | N-TAMRA-Ser-Asp-Ser-Asp-Ser-Asp-Ser-Asp-Ser-C | 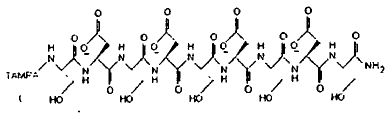 |
| 2DOS-12 | N-TAMRA-Asp-Asp-Asp-Asp-Asp-Asp-Asp-Asp-Asp-C | 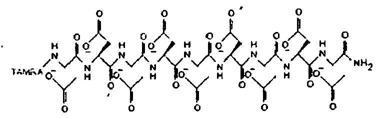 |
| 2DOS-13 | N-TAMRA-Asp-Norl eu-Asp-Norleu-As p-Norleu-Asp-C | 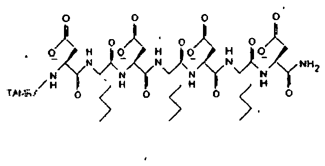 |
| 2DOS-14 | N-TAMRA-Asp-Phe-Asp-Phe-Asp-Phe-Asp-C | 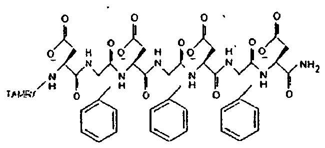 |
| 2DOS-15 | N-TAMRA-Asp-Ser-Asp-Ser-Asp-Ser-Asp-C | 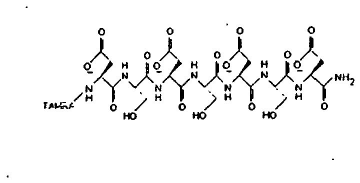 |
| 2DOS-16 | N-TAMRA-Asp-Asp-Asp-Asp-Asp-Asp-Asp-C | 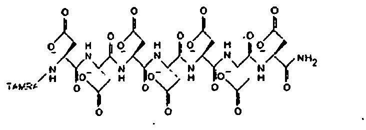 |

Peptides were synthesised on a 2 µmol scale using standard FMOC chemistry (Alta Bioscience) and were dissolved to 10 µM in 50% (v/v) aqueous acetonitrile

The retention of peptides 2DOS-1 to 2D.OS-16 on a hydrophobic surface (the wells of a polypropylene microtitre plate) was then investigated.

10 mM tris-HCl (pH 8.0) in 50% (v/v) aqueous acetonitrile was used as the solvent for the peptides and for TAMRA.

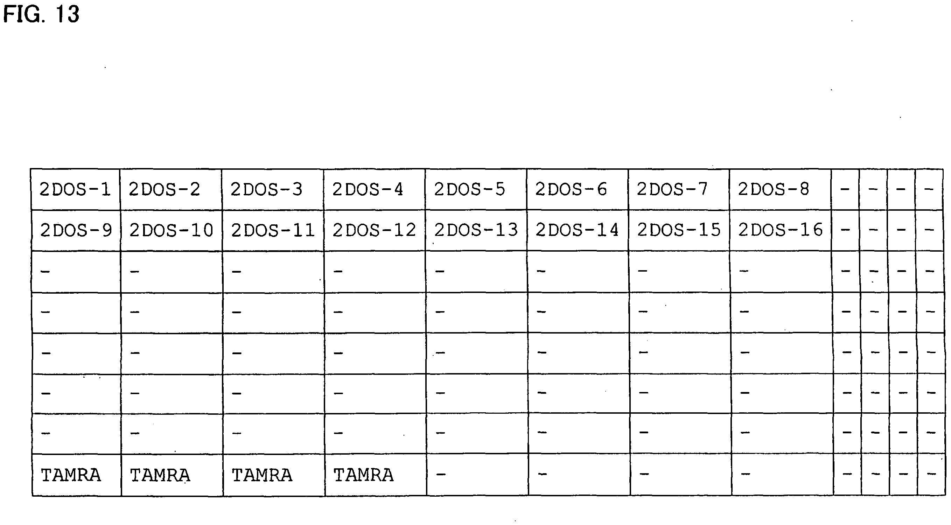

100 µl aliquots of 10 µM peptides 2DOS-1 to 2DOS-16 and 10 µM TAMRA were placed in the wells of a Costar microtitre plate as shown in Figure 13:

The microtitre plate was imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at +3 mm and the sample was pressed during scanning.

The peptides were allowed to evaporate to dryness overnight in the dark and the microtitre plate was again scanned as described above.

The wells were then washed ten times with 250 µl of water.

The residual surface-bound peptides were finally resuspended in 100 µl of 10 mM tris-HCl (pH 8.0) in 50% (v/v) aqueous acetonitrile and the microtitre plate was again scanned as described above.

The fluorescence images were analysed using ImageQuant TL v2003.03 (Amersham Biosciences).

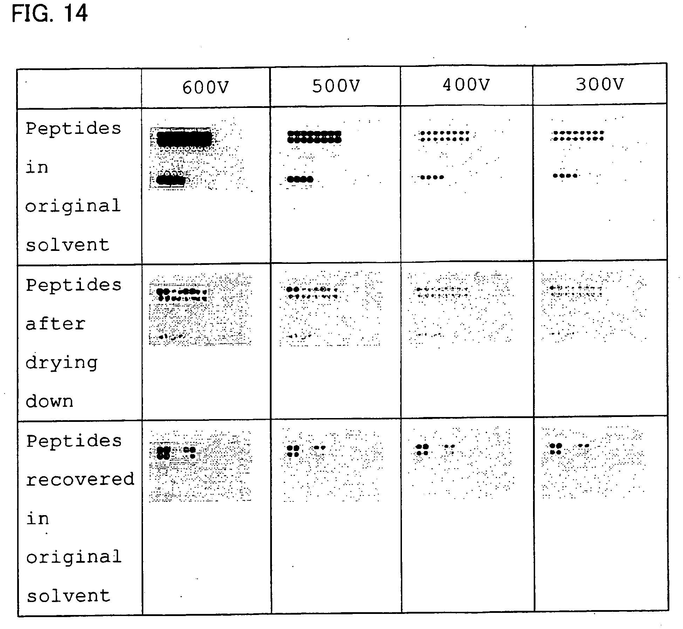

The fluorescence images of the microtitre plate scanned at PMT voltages of 600V, 500V, 400V, and 300V at the three stages of the experiment are shown in Figure 14:

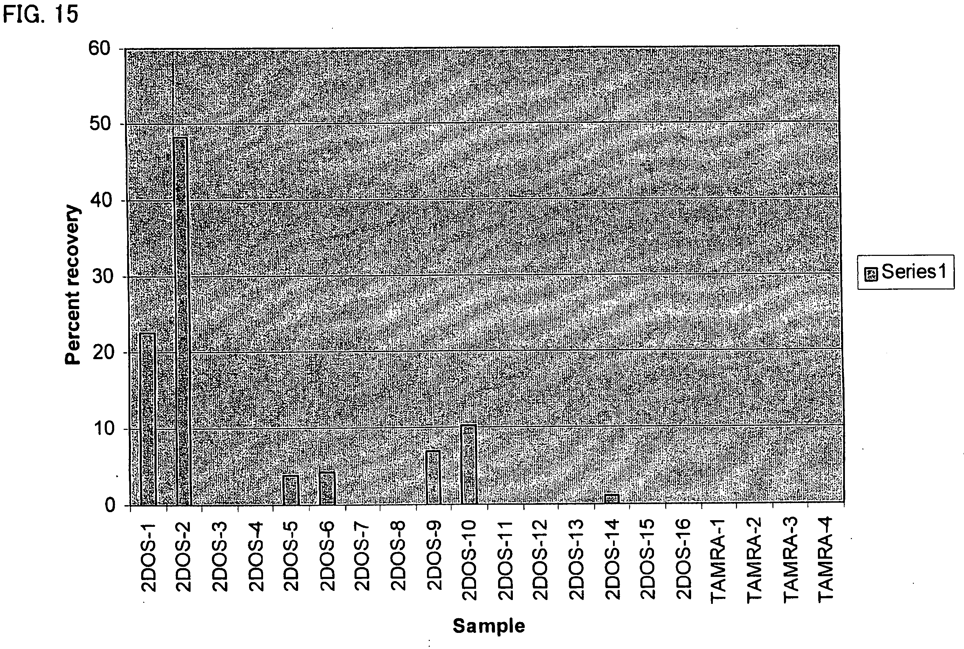

Quantification data (using the data from the 400V scan) is given in Table 2 and shown graphically in Figure 15.

**Table 2**

| Peptide | Initial fluorescence (x10³) | Recovered fluorescence (x10³) | Percent recovery |
|---|---|---|---|
| 2DOS-1 | 33,015 | 7,459 | 23 |
| 2DOS-2 | 32, 492 | 15,704 | 48 |
| 2DOS-3 | 32,913 | 8 | 0 |
| 2DOS-4 | 32,313 | 0 | 0 |
| 2DOS-5 | 32,473 | 1,270 | 4 |
| 2DOS-6 | 33,853 | 1, 455 | 4 |
| 2DOS-7 | 29,134 | 0 | 0 |
| 2DOS-8 | 33,615 | 3 | 0 |
| 2DOS-9 | 34,587 | 2,382 | 7 |
| 2DOS-10 | 28,479 | 2,889 | 10 |
| 2DOS-11 | 26,860 | 0 | 0 |
| 2DOS-12 | 26,433 | 1 | 0 |
| 2DOS-13 | 26,181 | 25 | 0 |
| 2DOS-14 | 28,071 | 283 | 1 |
| 2DOS-15 | 30,845 | 2 | 0 |
| 2DOS-16 | 30,335 | 3 | 0 |

The results show that phenylalanyl residues lead to greater retention than norleucyl residues. They also show that peptides with five hydrophobic 'anchor residues' are retained better than equivalent peptides with three hydrophobic 'anchor residues'. Changing the 'ligand-binding' residues from aspartyl, alanyl, seryl, and lysyl to a run of four aspartyl residues leads to a drop in retention on the polypropylene surface..

Further experiments were undertaken to investigate the retention of peptides P1-1 to P1-5 and P2-1 to P2-2, shown in Table 3, on a polypropylene surface.

**Table 3**

| Peptide | Sequence |
|---|---|
| P1-1 | TAMRA-F-G-F-S-F-A-F-D-F-G-F |
| P1-2 | TAMRA-F-G-F-G-F-S-F-A-F-D-F-G-F |
| P1-3 | TAMRA-F-G-F-G-F-G-F-S-F-A-F-D-F-G-F |
| P1-4 | TAMRA-F-G-F-G-F-G-F-G-F-S-F-A-F-D-F-G-F |
| P1-5 | TAMRA-F-G-F-G-F-G-F-G-F-G-F-S-F-A-F-D-F-G-F |
| P2-1 | TAMRA-G-S-F-A-F-D-F |
| P2-2 | TAMRA-G-S-G-A-F-D-F |

The polypropylene sheet was wiped with 50% (v/v) aqueous acetonitrile prior to use.

8x replicate 20 nl volumes of 1 µM peptides P1-1 to P1-5 and P2-1 to P2-2 and TAMRA in dimethyl sulphoxide (DMSO) were dispensed at 1 mm spacing to a 3"x1"x1 mm polypropylene sheet using the Piezorray system (PerkinElmer LAS). 500 drops were pre-dispensed using the 'side shoot' option and the tuning was set to 72V for 30µs.

The slide was imaged at 10 µm resolution on a Typhoons Trio Plus variable mode imager (Amersham Biosciences) with the green (532 nm) laser and the 580 BP 30 filter at a PMT voltage of 600 V and at normal sensitivity. The scan height was set at the platen and the samples were pressed during scanning. The fluorescence image was analysed using ImageQuant TL v2003.03 (Amersham Biosciences).

The lower half of the slide (containing the test array) was then washed in 100 ml of 1 M NaCl containing 10 mM 'tris-HCl (pH 8.0) for one minute and were re-scanned as described above.

The same half of the slide was then washed for a second time in 100 ml of 1 M NaCl containing 10 mM tris-HCl (pH 8.0) for 30 minutes and re-scanned as described above.

The same half of the slide was then washed for a third time in 100 ml of waster for 30 minutes and re-scanned as described above.

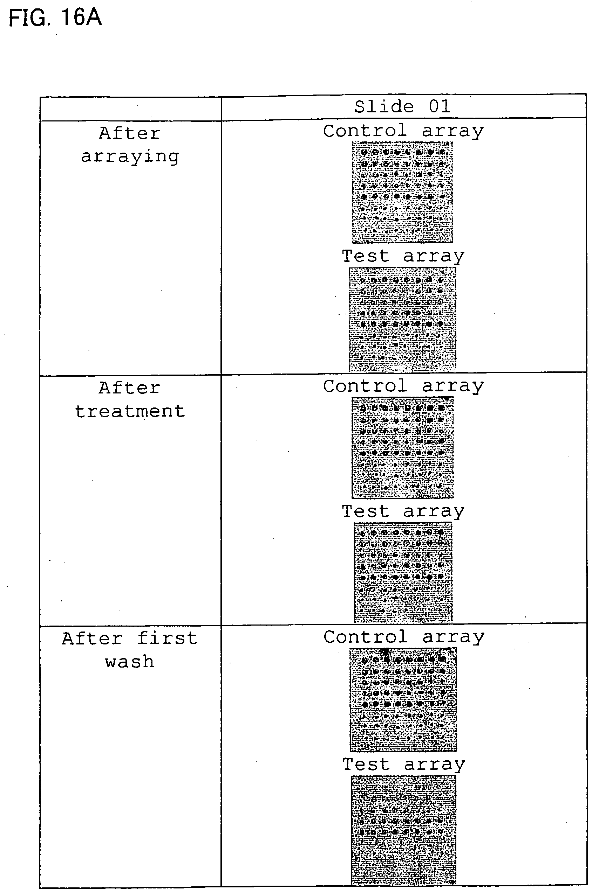

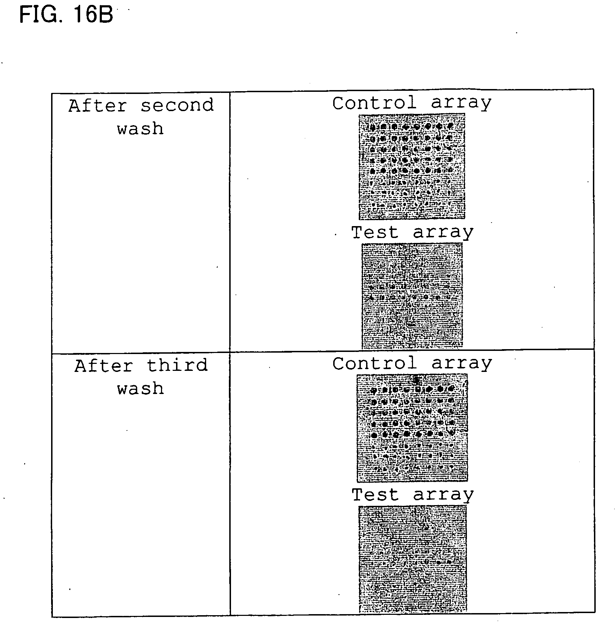

The fluorescence images for the various arrays are shown in Figure 16A,B.

The fluorescence values for the various arrayed peptides shown in Figure 16A,B are shown in Tables 4 - 6 below:

After first wash:

**Table 4a**

| Control array | | |
|---|---|---|
| Peptide | Average fluorescence | Corrected signal |
| P1-1 | 244,263,013 | 97,571,391 |
| P1-2 | 200,280,129 | 53,588,457 |
| P1-3 | 192,743,469 | 46,051,796 |
| P1-4 | 187,287,630 | 40,595,958 |
| P1-5 | 199,347,483 | 52,655,810 |
| | | |
| Average slide background = | 146, 691, 673 | |

**Table 4b**

| Test array | | | |
|---|---|---|---|
| Peptide | Average fluorescence | Corrected signal | Percentage recovery |
| P1-1 | 157, 624, 537 | 3, 126, 578 | 3 |
| P1-2 | 170,078,218 | 15, 580, 260 | 29 |
| P1-3 | 171,197,973 | 16,700,014 | 36 |
| P1-4 | 189,823,310 | 35,325,352 | 87 |
| P1-5 | 201,991,732 | 47,493,774 | 90 |
| | | | |
| Average slide background = | 154,497,959 | | |

After second wash:

**Table 5a**

| Control array | | |
|---|---|---|
| Peptide | Average fluorescence | Corrected signal |
| P1-1 | 225, 863, 933 | 88, 584, 083 |
| P1-2 | 196, 080, 891 | 58, 801, 042 |
| P1-3 | 187, 310, 655 | 50, 030, 806 |
| P1-9 | 179, 942, 418 | 42, 662, 569 |
| P1-5 | 190, 847, 825 | 53, 567, 975 |
| | | |
| Average slide background = | 137, 279, 849 | |

**Table 5b**

| Test array | | | |
|---|---|---|---|
| Peptide | Average fluorescence | Corrected signal | Percentage recovery |
| P1-1 | 127, 216, 792 | -5, 230, 512 | -6 |
| P1-2 | 135, 695, 639 | 3, 248, 336 | 6 |
| P1-3 | 148, 725, 456 | 16, 278, 152 | 33 |
| P1-4 | 159, 100, 527 | 26, 653, 223 | 62 |
| P1-5 | 167, 865, 473 | 35, 418, 169' | 66 |
| | | | |
| Average slide background = | 132,947,303 | | |

After third wash:

**Table 6a**

| Control array | | |
|---|---|---|
| Peptide | Average fluorescence | Corrected signal |
| P1-1 | 218, 342, 010 | 89, 434, 769 |
| P1-2 | 185, 727, 868 | 51, 820, 628 |
| P1-3 | 184, 219, 147 | 50, 311, 907 |
| P1-4 | 176, 102, 987 | 42, 195, 247 |
| P1-5 | 183, 492, 293 | 49, 585, 053 |
| | | |
| Average slide background = | 133, 907, 240 | |

**Table 6b**

| Test array | | | |
|---|---|---|---|
| Peptide | Average fluorescence | Corrected signal | Percentage recovery |
| P1-1 | 124, 941, 941 | 644, 716 | 1 |
| P1-2 | 126, 193, 156 | 1, 895, 931 | 4 |
| P1-3 | 125, 717, 642 | 1, 420, 417 | 3 |
| P1-4 | 135, 681, 173 | 11, 383, 947 | 27 |
| P1-5 | 147, 812, 043 | 23, 514, 817 | 47 |
| | | | |
| Average slide background = | 124,297,225 | | |

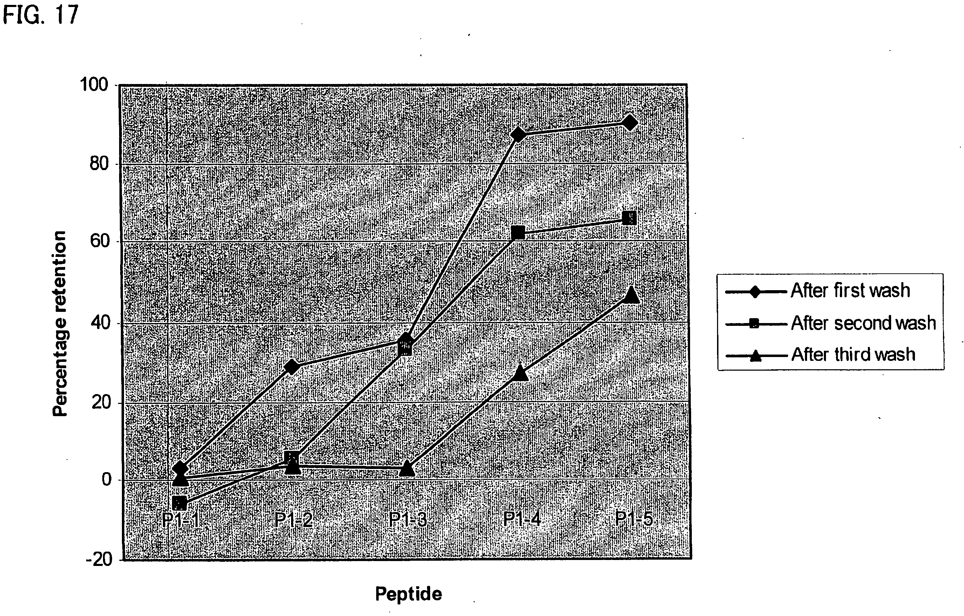

These results are shown graphically in Figure 17.

The figures clearly show that there is a gradient of increasing retention for the peptides correlated to increasing peptide chain length. As can be clearly seen, Peptide P1-5 which has a chain length of 19 amino acids has the highest retention.

### Example 3

The pH resistance of peptide 2DOS-2 (see above) deposited onto a polypropylene hydrophobic surface was investigated:

Twelve 50 µl aliquots of peptide 2DOS-2 in 10 mM tris-HCl (pH 8.0) in 50% (v/v) aqueous acetonitrile were dried down in the wells of a Costar microtitre plate. "

The peptide samples were allowed to evaporate to dryness in the dark.

The dried peptide samples in the first eleven wells were incubated with 200 µl of 100 mM phosphate buffer for 30 minutes at room temperature according to the scheme shown in Table 3:

**Table 7**

| Well | µl of 100 mM NaH₂PO₄ | µl of 100 mM Na₂HPO₄ | Observed pH |
|---|---|---|---|
| 1 | 1,000 | 0 | 4.51 |
| 2 | 900 | 100 | 5.65 |
| 3 | 800 | 200 | 6.02 |
| 4 | 700 | 300 | 6.25 |
| 5 | 600 | 400 | 6.47 |
| 6 | 500 | 500 | 6.62 |
| 7 | 400 | 600 | 6.79 |
| 8 | 300 | 700 | 6.98 |
| 9 | 200 | 800 | 7.18 |
| 10 | 100 | 900 | 7.47 |
| 11 | 0 | 1,000 | 8.52 |

All supernatants were pipetted off and the residual surface-bound peptides in all twelve wells were finally resuspended in 50 µl of 10 mM tris-HCl (pH 8.0) in 50% (v/v) aqueous acetonitrile and the microtitre plate was imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at +3 mm and the sample was pressed during scanning.

The fluorescence images were analysed using ImageQuant TL v2003.03 (Amersham Biosciences).

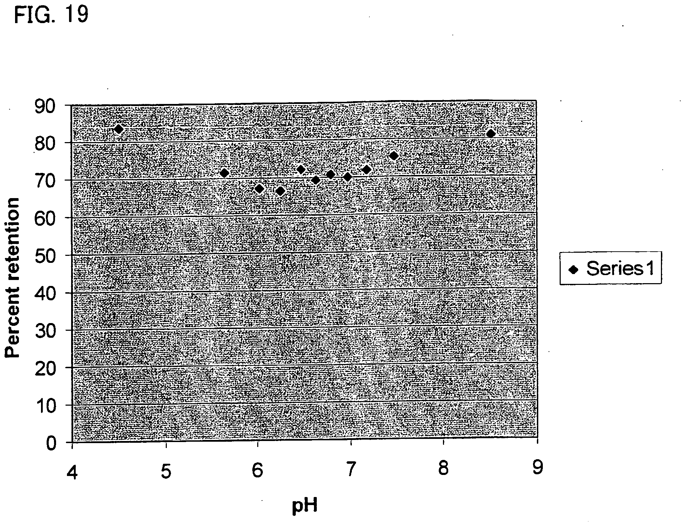

Fluorescence images showing the pH resistance of 2DOS-2 deposited on polypropylene are shown in Figure 19:

Quantification data for peptide 2DOS-2 retention (using data from the 300V scan) are given in Table 8 and graphically in Figure 19:

**Table 8**

| Well | Wash pH | Untreated fluorescence | Retained fluorescence | Percent retention |
|---|---|---|---|---|
| 1 | 4.51 | 815,706 | 681,015 | 83 |
| 2 | 5.65 | 815,706 | 582,482 | 71 |
| 3 | 6.02 | 815,706 | 548,329 | 67 |
| 4 | 6.25 | 815,706 | 542,595 | 67 |
| 5 | 6.47 | 815,706 | 589,528 | 72 |
| 6 | 6.62 | 815,706 | 566,496 | 69 |
| 7 | 6.79 | 815,706 | 576,655 | 71 |
| 8 | 6.98 | 815, 706 | 570,653 | 70 |
| 9 | 7.18 | 815,706 | 586,083 | 72 |
| 10 | 7.47 | 815,706 | 614,028 | 75 |
| 11 | 8.52 | '815,706 | 661,781 | 81 |

The results show that the retention of peptide 2DOS-2 on a polypropylene surface is therefore stable over a broad range of pH values, with maximal retention at low and high pH and minimal retention around pH 6.5.

### Example 4

The time-dependent persistence of peptide 2DOS-2 (see above) deposited onto a polypropylene hydrophobic surface in the presence of aqueous buffer was investigated as shown below:

Twelve 100 µl aliquots of 5 µM peptide 2DOS-2 in 5 mM tris-HCl (pH 8.0) in 75% (v/v) aqueous acetonitrile were dispensed to the wells of the top row of a Costar microtitre plate.

The peptide samples were allowed to evaporate to dryness in the dark.

The dried peptide samples in wells 1-10 were incubated with 250 µl of 1 M NaCl in 10 mM tris-HCl (pH 8.0) for the time indicated below at room temperature. All supernatants were pipetted up and down 8 times after incubation and the supernatants were then removed and placed in the wells of the bottom row of the microtitre plate.

The residual surface-bound peptides in all twelve wells were finally resuspended in 50 µl of 10 mM, tris-HCl (pH 8.0) in 50% (v/v) aqueous acetonitrile and the microtitre plates were imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at +3 mm and the sample was pressed during scanning.

The fluorescence images were analysed using ImageQuant TL v2003.03 (Amersham Biosciences).

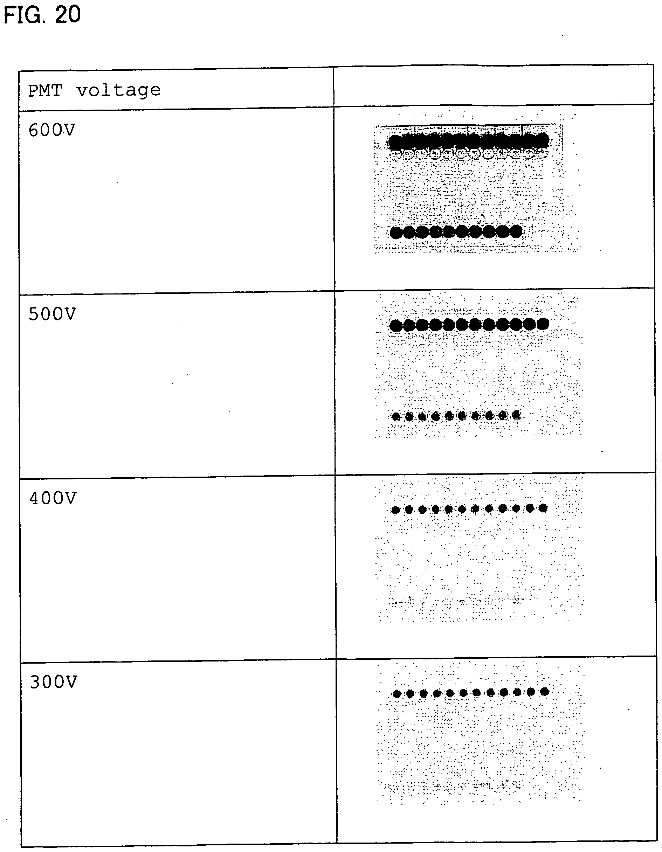

Fluorescence data for the time-dependent persistence of peptide 2DOS-2 deposited onto a polypropylene hydrophobic surface in the presence of aqueous buffer are shown in Figure 20:

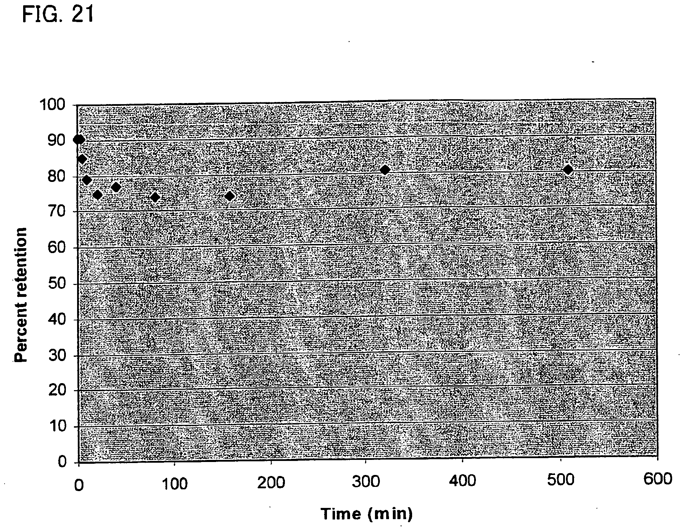

Quantification data for peptide 2DOS-2 retention (using data from the 300V scan) are shown in Table 9 and Figure 21:

**Table 9**

| Well | Minutes in 1 M NaCl / 10 mM tris-HCl (pH 8.0) | Untreated fluorescence (average) | Retained fluorescence | Percent retention |
|---|---|---|---|---|
| 1 | 0 | 1,000,613 | 904,211 | 90 |
| 2 | 2,5 | 1,000,613 | 902,082 | 90 |
| 3 | 5 | 1,000,613 | 847,767 | 85 |
| 4 | 10 | 1,000,613 | 792,427 | 79 |
| 5 | 20 | 1, 000, 613 | 749,522 | 75 |
| 6 | 40 | 1,000,613 | 769,659 | 77 |
| 7 | 80 | 1,000,613 | 740,227 | 74 |
| 8 | 160 | 1,000,613 | 739,600 | 74 |
| 9 | 320 | 1,000,613 | 805, 530 | 81 |
| 10 | 510 | 1,000,613 | 803,741 | 80 |

The results show that retention of peptide 2DOS-2 on a hydrophobic polypropylene surface is stable for extended periods of time in 1 M NaCl / 10 mM tris-HCl (pH 8.0).

### Example 5

The retention of peptides 2DOS-1 1 to 2DOS-16 (see above) on polypropylene wells of different geometries was investigated:

10 mM tris-HCl (pH 8.0) in 50% (v/v) aqueous acetonitrile was used as the solvent for the peptides and for TAMRA.

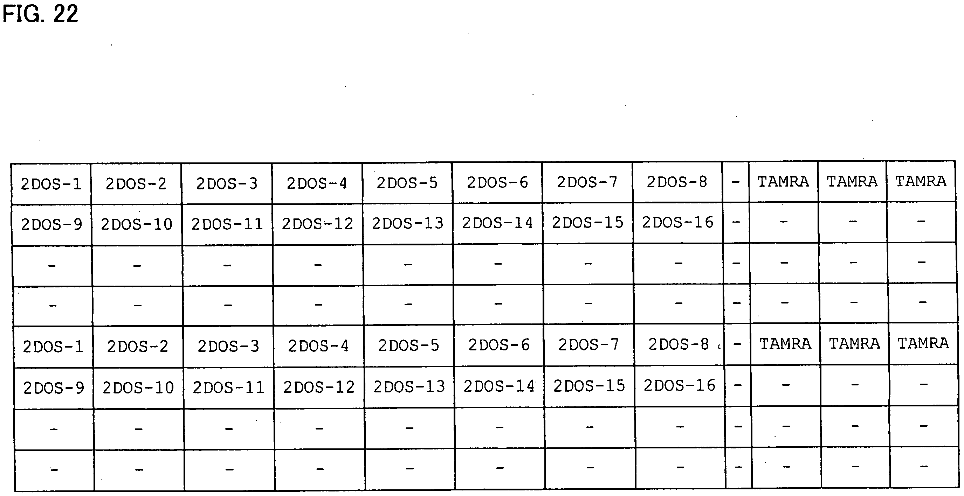

1 µl aliquots of 10 µM peptides 2DOS-1 to 2DOS-16 and 10 µM TAMRA were pipetted into the wells of a Costar V-bottomed polypropylene microtitre plate and a Greiner flat-bottomed polypropylene microtitre plate according to the following scheme as shown in Figure 22:

The peptide samples were allowed to evaporate to dryness in the dark.

The peptide samples in the top two rows of the microtitre plates were then incubated for 15 minutes at room temperature in 250 µl of 1 M NaCl in 10 mM tris-HCl (pH 8.0). After incubation, the wash buffer was pipetted up and down eight times in the well before removing the supernatant.

The washed and untreated peptide samples were then resuspended in 50 µl of 10 mM tris-HCl (pH 8.0) in 50% (v/v) aqueous acetonitrile.

The microtitre plates were imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at the platen and the sample was pressed during, canning.

The fluorescence images were analysed using ImageQuant TL v2003.03 (Amersham Biosciences).

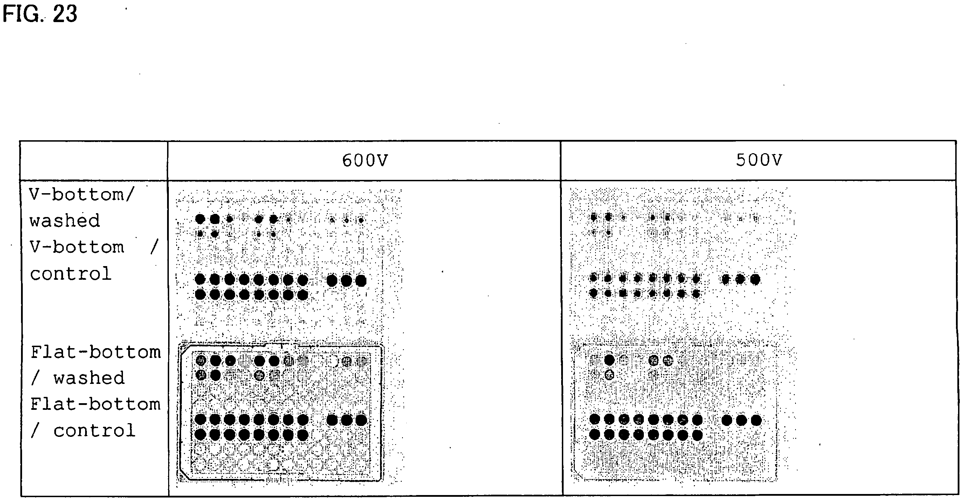

The fluorescence images of the plates scanned at PMT voltages of 600V and 500V are shown in Figure 23.

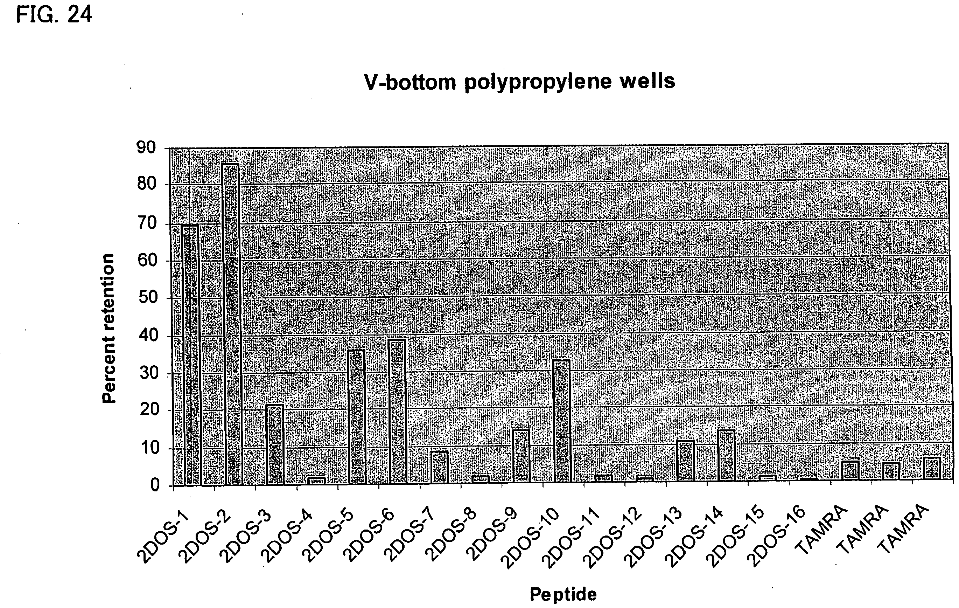

Quantification data for the V-bottom wells (using data from the 500V scan) are shown in Table 10 and Figure 24:

**Table 10**

| Peptide | Untreated fluorescence | Retained fluorescence | Percent retention |
|---|---|---|---|
| 2DOS-1 | 9,550,835 | 6,641,271 | 70 |
| 2DOS-2 | 9,495,183 | 8,127,309 | 86 |
| 2DOS-3 | 9,020,171 | 1,951,694 | 22 |
| 2DOS-4 | 8,265,596 | 159,080 | 2 |
| 2DOS-5 | 8,664,667 | 3,116,833 | 36 |
| 2DOS-6 | 9,141,290 | 3,527,116 | 39 |
| 2DOS-7 | 8, 674, 544 | 746,596 | 9 |
| 2DOS-8 | 10,130,734 | 202, 943 | 2 |
| 2DOS-9' | 11, 662, 691 | 1,608,482 | 14 |
| 2DOS-10 | 8,445,090 | 2,773,876 | 33 |
| 2DOS-11 | 9,705,293 | 192,272 | 2 |
| 2DOS-12 | 6, 777, 661 | 52,365 | 1 |
| 2DOS-13 | 7,623,227 | 806,022 | 11 |
| 2DOS-14 | 7, 641,246 | 1,018,179 | 13 |
| 2DOS-15 | 10,493,100 | 122,851 | 1 |
| 2DOS-16 | 9,782,527 | 44,420 | 0 |
| TAMRA | 12,610,993 | 640,684 | 5 |
| TAMRA | 15,392,751 | 686,863 | 4 |
| TAMRA | 17,471,866 | 998,320 | 6 |

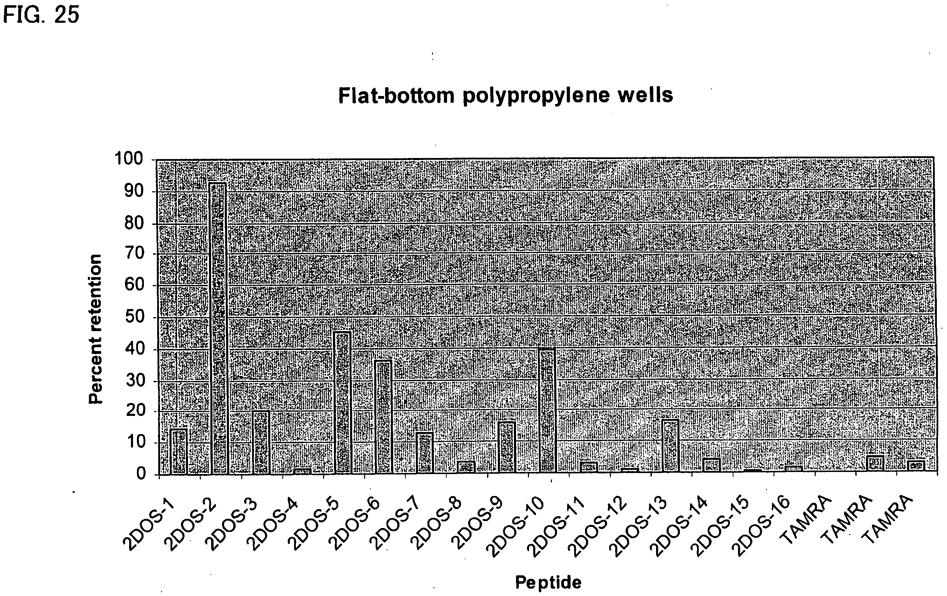

Quantification data for the flat-bottom wells (using data from the 500V scan) are shown in Table 11 and Figure 25:

**Table 11**

| Peptide | Untreated fluorescence | Retained fluorescence | Percent retention |
|---|---|---|---|
| 2DOS-1 | 14, 949, 396 | 2, 157, 129 | 14 |
| 2DOS-2 | 15, 820, 680 | 14, 665, 720 | 93 |
| 2DOS-3 | 14, 152, 875 | 2, 836, 015 | 20 |
| 2DOS-4 | 11, 885, 905 | 198, 507 | 2 |
| 2DOS-5 | 14, 337, 629 | 6, 492, 170 | 45 |
| 2DOS-6 | 14, 539,109 | 5, 256, 539 | 36 |
| 2DOS-7 | -10, 189, 473 | 1, 303, 195 | 13 |
| 2DOS-8 | 17, 576, 485 | 637, 350 | 4 |
| 2DOS-9 | 13, 456, 698 | 2, 148, 849 | 16 |
| 2DOS-10 | 13, 661, 609 | 5, 353, 630 | 39 |
| 2DOS-11 | 13, 960, 552 | 476, 318 | 3 |
| 2DOS-12 | 12, 982, 170 | 106, 741 | 1 |
| 2DOS-13 | 12, 935, 739 | 2, 162, 164, | 17 |
| 2DOS-14 | 15, 090, 582 | 670, 341 | 4 |
| 2DOS-15 | 15, 290, 885 | 104, 523 | 1 |
| 2DOS-16 | 15, 555, 465 | 229, 090 | 1 |
| TAMRA | 18, 396, 859 | 0 | 0 |
| TAMRA | 20, 662, 891 | 935, 664 | 5 |
| TAMRA | 20, 649, 165 | 678, 001 | 3 |

The results show that retention of peptides 2DOS-1 to 2DOS-16 on polypropylene wells of different geometries is comparable, indicating that retention is not dependent upon drying down in wells with a V-bottomed geometry.

### Example 6

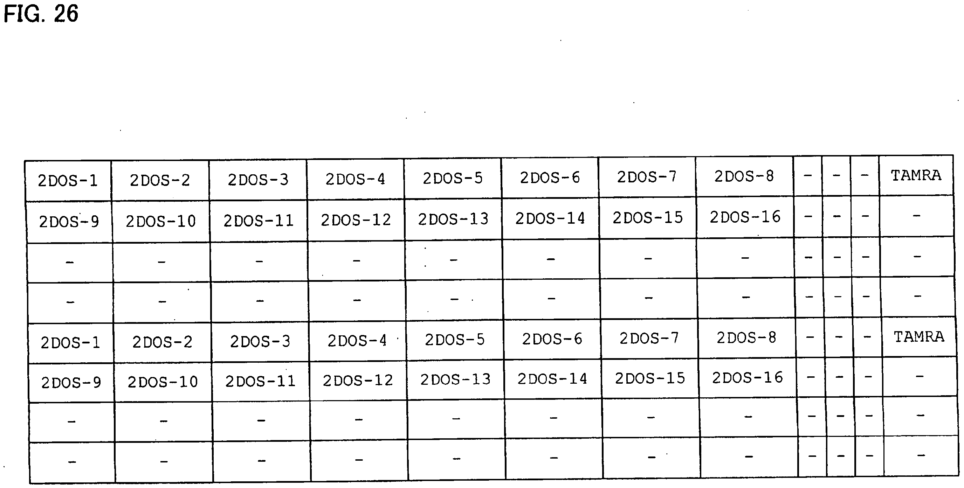

The retention of peptides 2DOS-1 to 2DOS-16 (see above) on polypropylene and polystyrene surfaces was compared:
5 mM tris-HCl (pH 8.0) in 75% (v/v) aqueous acetonitrile was used as the solvent for the peptides and for TAMRA.
20 µl aliquots of 5 µM peptides 2DOS-1 to 2DOS-16 and 5 µM TAMRA were pipetted into the wells of a Costar V-bottomed polypropylene microtitre plate, a Greiner V-bottomed polypropylene microtitre plate, and a Greiner V-bottomed polystyrene microtitre plate according to the scheme shown in Figure 26:

The peptide samples were allowed to evaporate to dryness in the dark.

The peptide samples in the top two rows of the microtitre plates were then incubated for 15 minutes at room temperature in 250 µl of 1 M NaCl in 10 mM tris-HCl (pH 8.0). After incubation, the wash buffer was pipetted up and down eight times in the well before removing the supernatant.

The washed and untreated peptide samples were then resuspended in 50 µl of 10 mM tris-HCl (pH 8.0) in 50% (v/v) aqueous acetonitrile.

The microtitre plates were imaged at 200 µm resolution on a Typhoon Trio Plus variable mode imager (Amersham Biosciences) with the green (532 nm) laser and the 580 BP 30 filter at the PMT voltages indicated below and at normal sensitivity. The scan height was set at +3 mm and the sample was pressed during scanning.

The fluorescence images were analysed using ImageQuant TL v2003.03 (Amersham Biosciences).

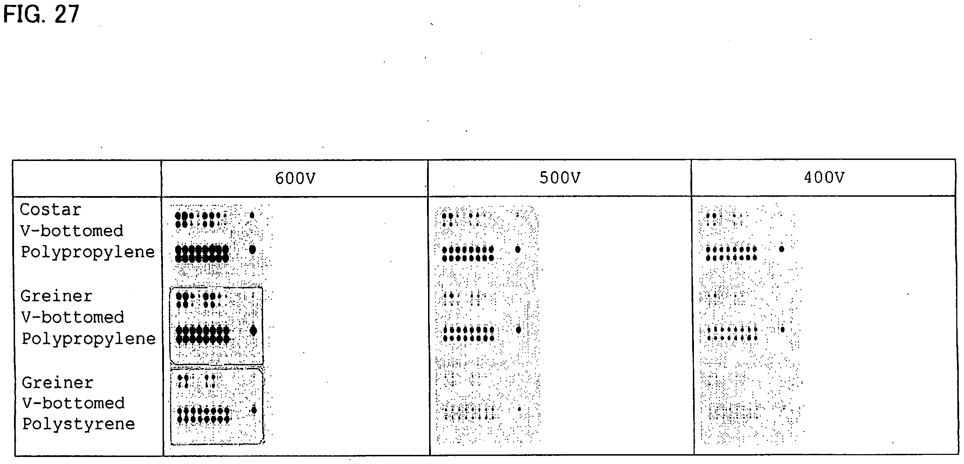

The fluorescence images of the slides scanned at PMT voltages of 600V, 500V, and 400V are shown in Figure 27:

Peptide samples in the upper half of the plates have been washed and peptide samples in the lower half of the plates are untreated.

Quantification data for the Costar polypropylene V-bottom wells (using data from the 400V scan) are given in Table 12:

**Table 12**

| Peptide | Untreated fluorescence. | Retained fluorescence | Percent retention |
|---|---|---|---|
| 2DOS-1 | 6, 458, 364 | 3, 366, 403 | 52 |
| 2DOS-2 | 5, 692, 134 | 5, 349, 601 | 94 |
| 2DOS-3 | 5, 660, 618 | 673, 940 | 12 |
| 2DOS-4 | 5, 661, 181 | 74, 193 | 1 |
| 2DOS-5 | 5, 331, 763 | 1, 651, 850 | 31 |
| 2DOS-6 | 5, 733, 046 | 1, 256, 204 | 22 |
| 2DOS-7 | 5, 139, 578 | 326, 328 | 6 |
| 2DOS-8 | 6, 587, 741 | 93, 455 | 1 |
| 2DOS-9 | 7, 102, 251 | 1, 184, 648 | 17 |
| 2DOS-10 | 6, 043, 214 | 1, 939, 375 | 24 |
| 2DOS-11 | 5, 327, 435 | 102, 040 | 2 |
| 2DOS-12 | 4, 432, 090 | 18, 180 | 0 |
| 2DOS-13 | 4, 886, 617 | 387, 738 | 8 |
| 2DOS-14 | 5, 331, 894 | 595, 959 | 11 |
| 2DOS-15 | 6, 488, 130 | 50, 584 | 1 |
| 2DOS-16 | 5, 387, 850 | 16, 194 | 0 |
| TAMRA | 11, 786, 211 | 386, 629 | 3 |

Quantification data for the Greiner polypropylene V-bottom wells (using data from the 400V scan) are given in Table 13:

**Table 13**

| Peptide | Untreated fluorescence | Retained fluorescence | Percent retention |
|---|---|---|---|
| 2DOS-1 | 3, 328, 960 | 1, 235, 460 | 37 |
| 2DOS-2 | 3, 512, 023 | 2, 731, 827 | 78 |
| 2DOS-3 | 3, 583, 352 | 342, 889 | 10 |
| 2DOS-4 | 3, 937, 734 | 50, 669 | 1 |
| 2DOS-5 | 3, 817, 250 | 1, 048, 978 | 27 |
| 2DOS-6 | 3, 889, 995 | 849, 582 | 22 |
| 2DOS-7 | 3, 751, 280 | 205, 430 | 5 |
| 2DOS-8 | 3, 903, 470 | 80, 770 | 2 |
| 2DOS-9 | 3, 621, 913 | 439, 252 | 12 |
| 2DOS-10 | 3, 043, 118 | 654, 623 | 22 |
| 2DOS-11 | 3, 510, 896 | 86, 818 | 2 |
| 2DOS-12 | 3, 235, 590 | 20, 175 | 1 |
| 2DOS-13 | 3, 635, 229 | 378, 287 | 10 |
| 2DOS-14 | 3, 612, 113 | 461, 468 | 13 |
| 2DOS-15 | 4, 771, 265 | 65, 142 | 1 |
| 2DOS-16 | 3, 813, 060 | 25, 885 | 1 |
| TAMRA | 6, 199, 206 | 64, 916 | 1 |

Quantification data for the Greiner polystyrene V-bottom wells (using data from the 400V scan) are given in Table 14:

**Table 14**

| Peptide | Untreated fluorescence | Retained fluorescence | Percent retention |
|---|---|---|---|
| 2DOS-1 | 640, 145 | 225, 686 | 35 |
| 2DOS-2 | 614, 203 | 392, 422 | 64 |
| 2DOS-3 | 744, 747 | 37, 388 | 5 |
| 2DOS-4 | 783, 885 | 6, 714 | 1 |
| 2DOS-5 | 745, 029 | 171, 500 | 23 |
| 2DOS-6 | 666, 352 | 167, 118 | 25 |
| 2DOS-7 | 706, 200 | 15, 766 | 2 |
| 2DOS-8 | 842, 984 | 5, 071 | 1 |
| 2DOS-9 | 626, 304 | 62, 023. | 10 |
| 2DOS-10 | 602, 771 | 100, 265 | 17 |
| 2DOS-11 | 675, 269 | 7, 365 | 1 |
| 2DOS-12 | 684, 832 | 3, 788 | 1 |
| 2DOS-13 | 708, 162 | 62, 590 | 9 |
| 2DOS-14 | 860, 811 | 75, 304 | 9 |
| 2DOS-15 | 868, 334 | 5, 116 | 1 |
| 2DOS-16 | 789, 858 | 3, 864 | 0 |
| TAMRA | 1, 051, 154 | 10, 004 | 1 |

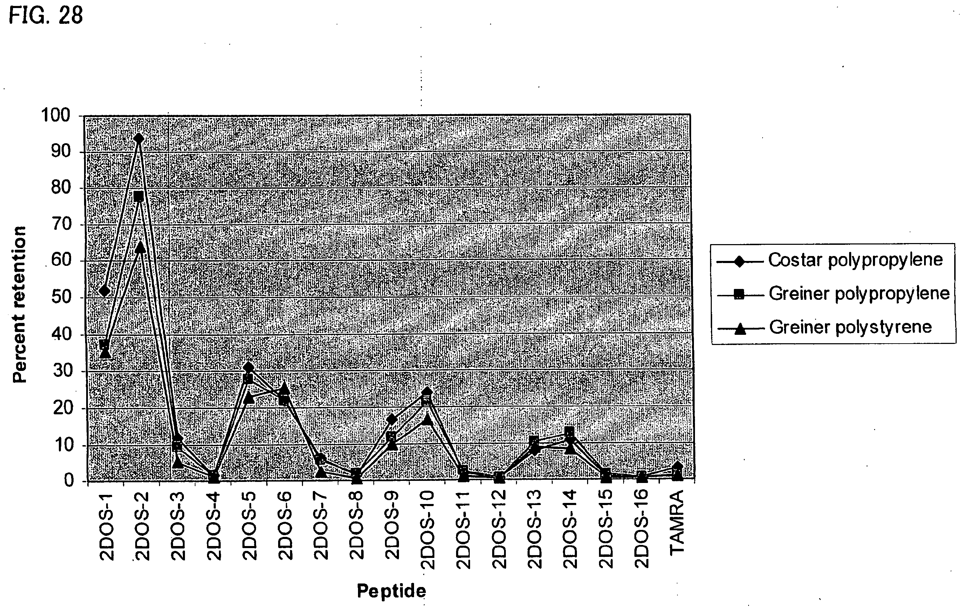

These data are shown graphically in Figure 28:

The results show that comparable peptide behaviour is seen on all three surfaces, demonstrating that retention is a sequence-specific property of the peptides rather than a property that is peculiar to one particular plastic surface.

### Example 7

Four peptides were synthesised that contain a 'surface-binding face' consisting of seven phenylalanyl residues. These peptides also contain a central region consisting of charged and uncharged residues and a variable penultimate residue. The variable penultimate residue was alanyl, seryl, cysteiyl, or nitropyridylthio activated cysteiyl.

An additional four TAMRA-labelled fluorescent peptides were also synthesised that contain an N-terminal TAMRA fluorophore attached to a glycyl residue that is attached to a variable C-terminal residue. The variable C-terminal residue was alanyl, seryl, cysteiyl, or nitropyridylthio activated cysteiyl.

A mixture of the 5-TAMRA and 6-TAMRA isomers as shown in Example 1 was used for the labelling.

The full set of eight peptides is shown in Tables 15 and 16:

**Table 15**

| Peptide | Sequence | Structure |
|---|---|---|
| SB-1 | N-Phe-Gly-Phe-Lys-Phe-Gly-Phe-Asp-Phe-Gly-Ph e-Ala-Phe-C | 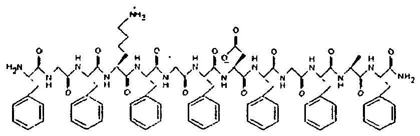 |
| SB-2 | N-Phe-Gly-Phe-Lys-Phe-Gly-Phe-Asp-Phe-Gly-Ph e-Ser-Phe-C | 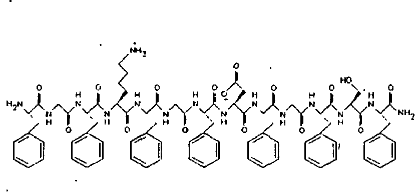 |
| SB-3 | N-Phe-Gly-Phe-Lys-Phe-Gly-Phe-Asp-Phe-Gly-Ph e-Cys-Phe-C | 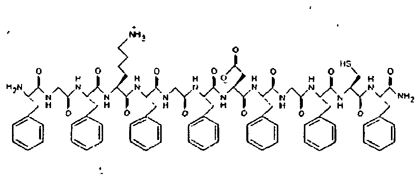 |
| SB-4 | N-Phe-Gly-Phe-Lys-Phe-Gly-Phe-Asp-Phe-Gly-Ph e-CysSNP-Phe-C | 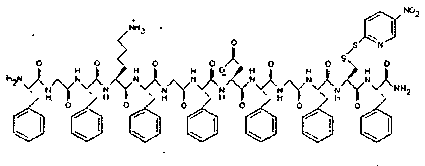 |

**Table 16**

| Peptide | Sequence | Structure |
|---|---|---|
| TLSP-1 | N-TAMRA-Gly-Ala-C | 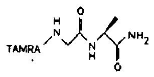 |
| TLSP-2 | N-TAMRA-Gly-Ser-C | 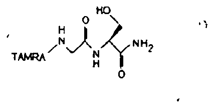 |
| TLSP-3 | N-TAMRA-Gly-Cys-C | 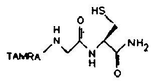 |
| TLSP-4 | N-TAMRA-Gly-CysSNP-C | 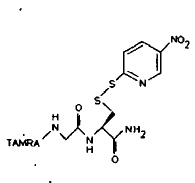 |

The peptides SB-1 to SB-4 and TLSP-1 to TLSP-4 were used in order to investigate dimer formation.

Two different protocols were used. In the 'liquid phase' protocol, The SB peptides were dried down onto a polypropylene surface. The TLSP peptides were then added in aqueous solution prior to washing the wells and assaying for retained fluorescent material.

In the 'co-drying' protocol, The SB peptides were mixed with the TLSP peptides and both were then dried down together onto a polypropylene surface prior to washing the wells and assaying for retained fluorescent material.

A further protocol in which the SB peptides are mixed with the TLSP peptides in aqueous solution and allowed to react to produce peptide dimers which are then dried down onto a polypropylene surface could easily be achieved by one skilled in the art.

In the 'liquid phase' protocol, 50 µl of 10 µM peptides SB-1 to SB-4 in 1 mM NaH₂PO₄ in 50% (v/v) aqueous acetonitrile were added the wells of a Costar V-bottomed microtitre plate according to the scheme shown in Table 17:

**Table 17**

| | | | | | | | | | | | |
|---|---|---|---|---|---|---|---|---|---|---|---|
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |

The samples were dried down overnight in the dark.

50 µl of 100 µM peptides TLSP-1 to TLSP-4 in 10 mM NaH2PO4 were then added to the wells according to the scheme shown in Table 18:

**Table 18**

| | | | | | | | | | | | |
|---|---|---|---|---|---|---|---|---|---|---|---|
| TLSP-1 | TLSP-1 | TLSP-1 | TLSP-1 | TLSP-1 | - | - | - | - | - | - | - |
| TLSP-2 | TLSP-2 | TLSP-2 | TLSP-2 | TLSP-2 | - | - | - | - | - | - | - |
| TLSP-3 | TLSP-3 | TLSP-3 | TLSP-3 | TLSP-3 | - | - | - | - | - | - | - |
| TLSP-4 | TLSP-4 | TLSP-4 | TLSP-4 | TLSP-4 | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |

The samples were incubated at room temperature for one hour in the dark. The supernatants were removed and the wells were washed twice with 200 µl of 1 M NaCl in 10 mM tris-HCl (pH 8.0). 50 µl of 10 mM tris-HCl (pH 8.0) in 50% (v/v) aqueous acetonitrile was finally added to the wells.

The microtitre plate was imaged at 200 µm resolution on a Typhoon Trio Plus variable mode imager (Amersham Biosciences) with the green (532 nm) laser and the 580 BP 30 filter at a PMT voltage of 500 V and at normal sensitivity. The scan height was set at +3 mm and the sample was pressed during scanning. The fluorescence image was analysed using ImageQuant TL v2003.03 (Amersham Biosciences).

In the 'co-drying' protocol, 50 µl of 10 µM peptides SB-1 to SB-4 in 1 mM NaH₂PO₄ in 50% (v/v) aqueous acetonitrile were added to the wells of a Costar V-bottomed microtitre plate according to the scheme shown in Table 19:

**Table 19**

| | | | | | | | | | | | |
|---|---|---|---|---|---|---|---|---|---|---|---|
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| SB-1 | SB-2 | SB-3 | SB-4 | - | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |

50 µl of 100 µM peptides TLSP-1 to TLSP-4 in 1 mM NaH₂PO₄ in 50% (v/v) aqueous acetonitrile were then added to the wells according to the scheme shown in Table 20:

**Table 20**

| | | | | | | | | | | | |
|---|---|---|---|---|---|---|---|---|---|---|---|
| TLSP-1 | TLSP-1 | TLSP-1 | TLSP-1 | TLSP-1 | - | - | - | - | - | - | - |
| TLSP-2 | TLSP-2 | TLSP-2 | TLSP-2 | TLSP-2 | - | - | - | - | - | - | - |
| TLSP-3 | TLSP-3 | TLSP-3 | TLSP-3 | TLSP-3 | - | - | - | - | - | - | - |
| TLSP-4 | TLSP-4 | TLSP-4 | TLSP-4 | TLSP-4 | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |
| - | - | - | - | - | - | - | - | - | - | - | - |

The samples were dried down overnight in the dark.

The wells were then washed twice with 200 µl of 1 M NaCl in 10 mM tris-HCl (pH 8.0). 50 µl of 10 mM tris-HCl (pH 8.0) in 50% (v/v) aqueous acetonitrile was finally added to the wells.

The microtitre plate was imaged at 200 µm resolution on a Typhoon Trio Plus variable mode imager (Amersham Biosciences) with the green (532 nm) laser and the 580 BP 30 filter at a PMT voltage of 500 V and at normal sensitivity. The scan height was set at +3 mm and the sample was pressed during scanning. The fluorescence image was analysed using ImageQuant TL v2003.03 (Amersham Biosciences).

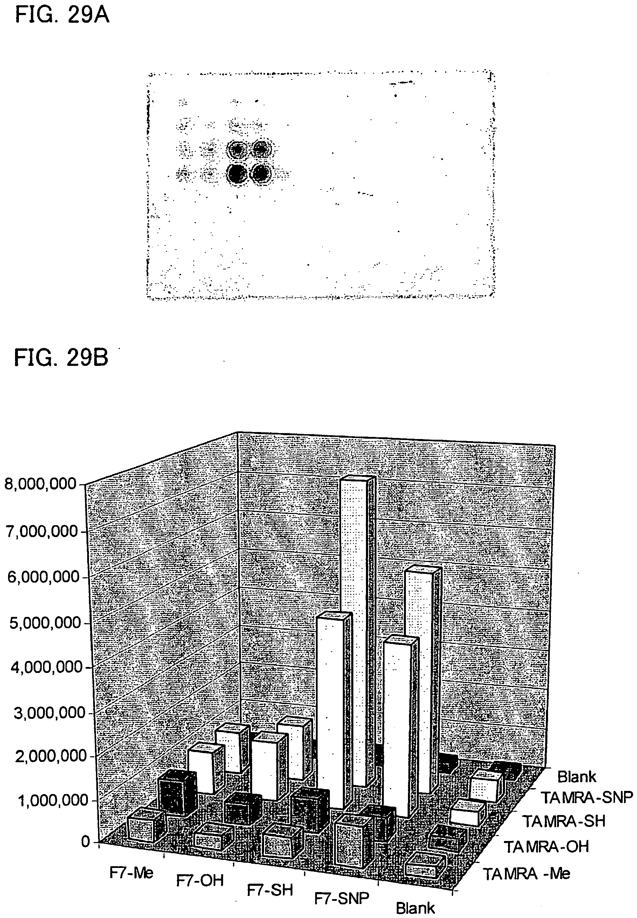

The fluorescence image for the microtitre plate from the experiment using the 'liquid phase' protocol is shown in Figure 29A.

The fluorescence data for the 'liquid phase' protocol are given in Table 21:

**Table 21**

| | SB-1 (F7-Me) | SB-2 (F7-OH) | SB-3 (F7-SH) | SB-4 (F7-SNP) | Blank |
|---|---|---|---|---|---|
| TLSP-1 (TAMRA -Me) | 505, 542 | 328, 552 | 494, 464 | 940, 493 | 250, 236 |
| TLSP-2 (TAMRA-OH) | 875, 810 | 495, 692 | 790, 731 | 574, 079 | 279, 409 |
| TLSP-3 (TAMRA-SH | 1, 024, 752 | 1, 449, 785 | 4, 531, 849 | 4, 101, 860 | 341, 250 |
| TLSP-4 (TAMRA-SNP) | 1, 021, 929 | 1, 357, 602 | 7, 434, 703 | 5, 378, 576 | 522, 053 |
| Blank | 266, 620 | 246, 461 | 285, 687 | 265, 120 | 203, 597 |

The results are shown graphically in Figure 29B:

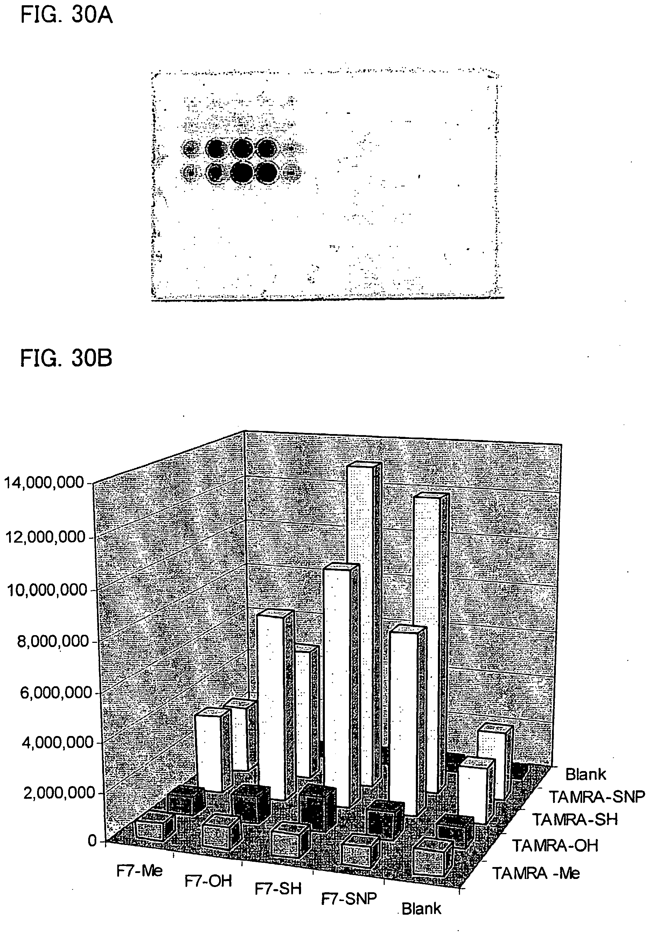

The fluorescence image for the microtitre plate from the experiment using the 'co-drying' protocol is shown in Figure 30A.

The fluorescence data for the 'co-drying' protocol are given in the following Table 22:

**Table 22**

| | SB-1 (F7-Me) | SB-2 (F7-OH) | SB-3 (F7-SH) | SB-4 (F7-SNP) | Blank |
|---|---|---|---|---|---|
| TLSP-1 (TAMRA -Me) | 719, 315 | 906, 087 | 919, 079 | 807, 652 | 970, 904 |
| TLSP-2 (TAMRA-OH) | 816, 019 | 1, 165, 325 | 1, 458, 222 | 1, 163, 929 | 892, 135 |
| TLSP-3 (TAMRA-SH | 3, 301, 896 | 7, 778, 287 | 9, 886, 083 | 7, 559, 276 | 2, 317, 125 |
| TLSP-4 (TAMRA-SNP) | 2, 862, 439 | 5, 555, 447 | 13, 506, 288 | 12, 389, 014 | 2, 876, 776 |
| Blank | 258, 401 | 295, 964 | 366, 184 | 378, 912 | 231, 826 |

The results are shown graphically in Figure 30B:

The yield of dimer is assayed by the retention of fluorescently labelled peptide which is conditional upon the presence of an unlabelled peptide that can bind to both the polypropylene surface and to the fluorescently labelled peptide.

For the 'liquid phase protocol' dimer yields are lower than for the 'co-drying' protocol but the chemical specificity for dimer formation is better. Maximal dimer formation is seen when the surface peptide possesses a free thiol group and the solution peptide possesses an S-nitropyridyl activated thiol group. Dimer formation is also observed when the surface peptide possesses an S-nitropyridyl activated thiol group and the solution peptide also possesses an S-nitropyridyl activated thiol group; when the surface peptide possesses a free thiol group and the solution peptide also possesses a free thiol group; and when the surface peptide possesses an S-nitropyridyl activated thiol group and the solution peptide possesses a free thiol group.

Free thiol coupling to free thiols may be due to simple aerobic oxidation, forming disulfide bonds. S-nitropyridyl activated thiol coupling to S-nitropyridyl activated thiols may be a result of incomplete thiol activation, leaving some free thiols able to 'react with the remaining S-nitropyridyl activated thiols, or some other mechanism.

Results for the 'co-drying' protocol mirror those described above. Dimer yields are generally higher with this method but non-specific binding is also higher. In particular, some reactivity is observed between a hydroxylated peptide attached to the surface and solution peptides containing both free thiol groups and S-nitropyridyl activated thiol groups.

### Example 8

In this example, peptide dimers are fabricated on a planar plastic surface using a Piezorray (PerkinElmer LAS) non-contact dispenser. The Piezorray (PerkinElmer LAS) is specifically designed for pipetting nanolitre volumes to dense arrays. Liquid volumes are controlled by a piezoelectric tip. The Piezorray system contains a source plate holder, an ultrasonic washbowl, a computer and monitor, and a bottle for system liquid.

Polypropylene sheet was obtained from SBA plastics (http://www.sba.co.uk/, Propylex Natural Polypropylene Sheet 2440 x 1220 x 1 mm) and was wiped with 50% (v/v) aqueous acetonitrile prior to use.

Six 10x10 arrays of 5nl of 100µM peptides SB-1 and SB-3 in 1mM NaH₂PO₄ in 50% (v/v) aqueous acetonitrile or solvent control were dispensed to 1 mm polypropylene sheet' cut to 3"x" using the Piezorray system according to the scheme shown in Table 23.

**Table 23**

| |
|---|
| 1 mM NaH₂PO₄ in 50% (v/v) aqueous acetonitrile |
| 1 mM NaH₂PO₄ in 50% (v/v) aqueous acetonitrile |
| Peptide SB-1 |
| Peptide SB-1. |
| Peptide SB-3 |
| Peptide SB-3 |

Six 10x10 arrays of 5 nl of 100 µM peptide TLSP-4 in either 1 mM NaH2PO4 in 50% (v/v) aqueous acetonitrile or in 1 mM NaH2PO4 in 50%, (v/v) aqueous acetonitrile and 10% (v/v) glycerol were then dispensed over the previous spots using the Piezorray system according to the scheme shown in Table 24:

**Table 24**

| |
|---|
| Peptide TLSP-4 |
| Peptide TLSP-4 in 10% glycerol |
| peptide TLSP-4 |
| Peptide TLSP-4 in 10% glycerol |
| Peptide TLSP-4 |
| Peptide TLSP-4 in 10% glycerol |

The samples were incubated at room temperature for 30 minutes in the dark. The slide was then washed in 100 ml of 50 mM naCl in 10 mM tris-HCl (pH 8.0) followed by running tap water over the slide for one minute.

The microtitre plate was imaged at 10 µm resolution on a Typhoon Trio Plus variable mode imager (Amersham Biosciences) with the green (532 nm) laser and the 580 BP 30 filter at a PMT voltage of 400 V and at normal sensitivity. The scan height was set at the platen and the sample was pressed during scanning. The fluorescence image was analysed using ImageQuant TL v2003.03 (Amersham Biosciences).

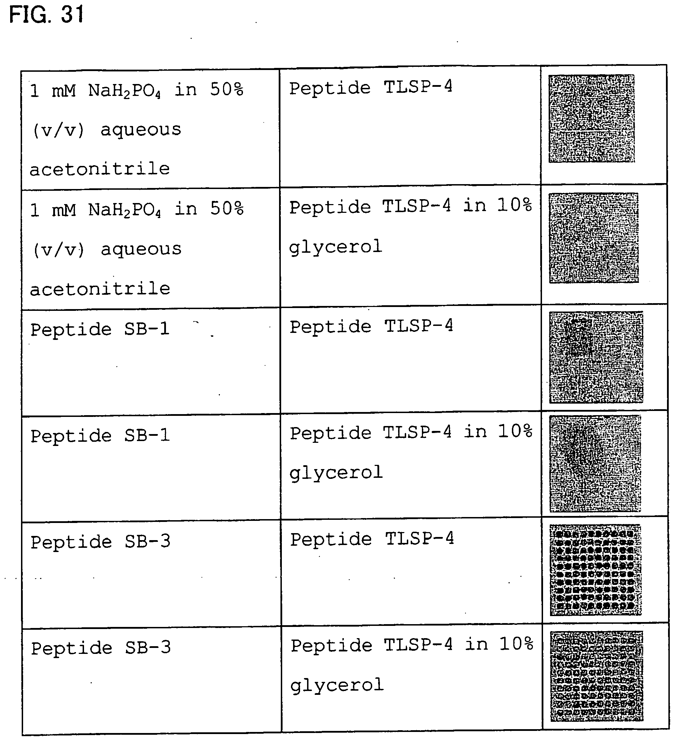

The fluorescence image for the polypropylene slide is shown in Figure 31:

The yield of dimer is assayed by the retention of fluorescently labelled peptide that is conditional upon the pretence of an unlabelled peptide that can bind to both the polypropylene surface and to the' fluorescently labelled peptide.

Dimer formation is therefore seen when the surface peptide possesses a free thiol group and the solution peptide possesses an S-nitropyridyl activated thiol group. The simple protocol (without glycerol to prevent evaporation) gives a higher yield of dimer.

### Example 9

In this example, twenty 256-clement microarrays of dimers comprising peptides L1-P1-1 to 16 and L1-P2-1 to 16 were fabricated in parallel.

1 mm polypropylene sheet was cut to 136 mm x 80mm, lightly abraded with glass paper, and wiped with 50% (v/v) aqueous acetonitrile prior to use.

18x 6 nl aliquots of P1 peptides were arrayed down the columns of the polypropylene slide at a spacing of 0.72 mm as indicated in Table 25, using the , Piezorray system (PerkinElmer LAS).

**Table 25**

| Column number | P1 peptide | Solvent |
|---|---|---|
| 1 | 2 µM P1-5 | 90% (v/v) DMSO, 1 mM tris-HCl (pH 8.0) |
| 2 | 20 uML1-P1-1 | 90% (v/v) DMSO, 1 mM tris-HCl (pH 8.0); 2 mM TCEP |
| 3 | 20 µM L1-P1-2 | 90% (v/v) DMSO, 1 mM tris-HCl (pH 8.0), 2 mM TCEP |
| 4 | 20 µM L1-P1-3 | 90% (v/v) DMSO, 1 mM tris-HCl (pH 8.0), 2 mM TCEP |
| 5 | 20 uML1-P1-4 | 90% (v/v) DMSO, 1 mM tris-HCl (pH 8.0), 2 mM TCEP |
| 6 | 20 uML1-P1-5 | 90% (v/v) DMSO, 1 mM tris-HCl (pH 8.0), 2 mM TCEP |
| 7 | 20 uML1-P1-6 | 90% (v/v) DMSO, 1 mM tris-HCl (pH 8.0), 2 mM TCEP |
| 8 | 20 µM L1-P1-7 | 90% (v/v) DMSO, 1 mM tris-HCl (pH 8.0), 2 mM TCEP |
| 9 | 20 µM L1-P1-8 | 90% (v/v) DMSO, 1 mM tris-HCl (pH 8.0), 2 mM TCEP |
| 10 | 20 µML1-P1-9 | 90% (v/v) DMSO, 1 mM tris-HCl (pH 8.0), 2 mM TCEP |
| 11 | 20 µML1-P1-10 | 90% (v/v) DMSO, 1 mM tris-HCl (pH 8.0), 2 mM TCEP |
| 12 | 20 µM L1-P1-11 | 90% (v/v) DMSO, 1 mM tris-HCl (pH 8.0), 2 mM TCEP |
| 13 | 20 µM L1-P1-12 | 90% (v/v) DMSO, 1 mM tris-HCl (pH 8.0), 2 mM TCEP |
| 14 | 20 µM L1-P1-13 | 90% (v/v) DMSO, 1 mM tris-HCl (pH 8.0), 2 mM TCEP |
| 15 | 20 µML1-P1-14 | 90% (v/v) DMSO, 1 mM tris-HCl (pH 8.0), 2 mM TCEP |
| 16 | 20µML1-P1-15 | 90% (v/v) DMSO, 1 mM tris-HCl (pH 8.0), 2 mM TCEP |
| 17 | 2oµmL1-P1-16 | 90% (v/v) DMSO, 1 mM tris-HCl (pH 8.0), 2 mM TCEP |
| 18 | 2 µM P1-5 | 90% (v/v) DMSO, 1 mM tris-HCl (pH 8.0) |

| | | |
|---|---|---|
| DMSO = dimethyl sulfoxide TCEP = tris (2-carboxyethyl) phosphine | | |

After sample evaporation, 18x 12 nl aliquots of L1-P2 peptides in 90% (v/v) DMSO, 1 mM tris-HCl (pH 8.0) were arrayed along the rows of the polypropylene slide at a spacing of 0.72 mm as indicated in Table 26, using the Piezorray system (PerkinElmer LAS).

**Table 26**

| Row number | Unlabelled P2 peptide | Labelled P2 peptides |
|---|---|---|
| 1 | - | 25 µM combined concentration of L1-P2-17/18/19/20 mixture |
| 2 | 50 µM L1-P2-1 | 25 µM combined concentration of L1-P2-17/18/19/20 mixture |
| 3 | 50 µM L1-P2-2 | 25 µM combined concentration of L1-P2-17/18/19/20 mixture |
| 4 | 50 µM L1-P2-3 | 25 µM combined concentration of L1-P2-17/18/19/20 mixture |
| 5 | 50 µM L1-P2-4 | 25 µM combined concentration of L1-P2-17/18/19/20 mixture |
| 6 | 50 µM L1-P2-5 | 25 µM combined concentration of L1-P2-17/18/19/20 mixture |
| 7 | 50 µM L1-P2-6 | 25 µM combined' concentration of L1-P2-17/18/19/20 mixture |
| 8 | 50 µM L1-P2-7 | 25 µM combined concentration of L1-P2-17/18/19/20 mixture |
| 9 | 50 µM L1-P2-8 | 25 µM combined concentration of L1-P2-17/18/19/20 mixture |
| 10 | 50 µM L1-P2-9 | 25 µM combined concentration of L1-P2-17/18/19/20 mixture |
| 11 | 50 µM L1-P2-10 | 25 µM combined concentration of L1-P2-17/18/19/20 mixture |
| 12 | 50 µM L1-P2-11 | 25 µM combined concentration of L1-P2-17/18/19/20 mixture |
| 13 | 50 µM L1-P2-12 | 25 µM combined concentration of L1-P2-17/18/19/20 mixture |
| 14 | 50µML1-P2-13 | 25 µM combined concentration of L1-P2-17/18/19/20 mixture |
| 15 | 50µML1-P2-14 | 25 µM combined concentration of L1-P2-17/18/19/20 mixture |
| 16 | 50µM L1-P2-15 | 25 µM combined concentration of L1-P2-17/18/19/20 mixture |
| 17 | 50 µM L1-P2-16 | 25 µM combined concentration of L1-P2-17/18/19/20 mixture |
| 18 | - | 25 µM combined concentration of L1-P2-17/18/19/20 mixture |

After sample evaporation, the slide was washed for' 10 minutes in 50 ml of 10 mM tris-HCl (pH 8.0) containing 0.1% (v/v) Tween-20.

The slide was imaged at 10 µm resolution on a Typhoon Trio Plus variable mode imager (Amersham Biosciences) with the green (532 nm) laser and the 580 BP 30 filter at a PMT voltage of 500V and at normal sensitivity. The scan height was set at the platen. The fluorescence image was analysed using ImageQuant TL v2003.03 (Amersham Biosciences).

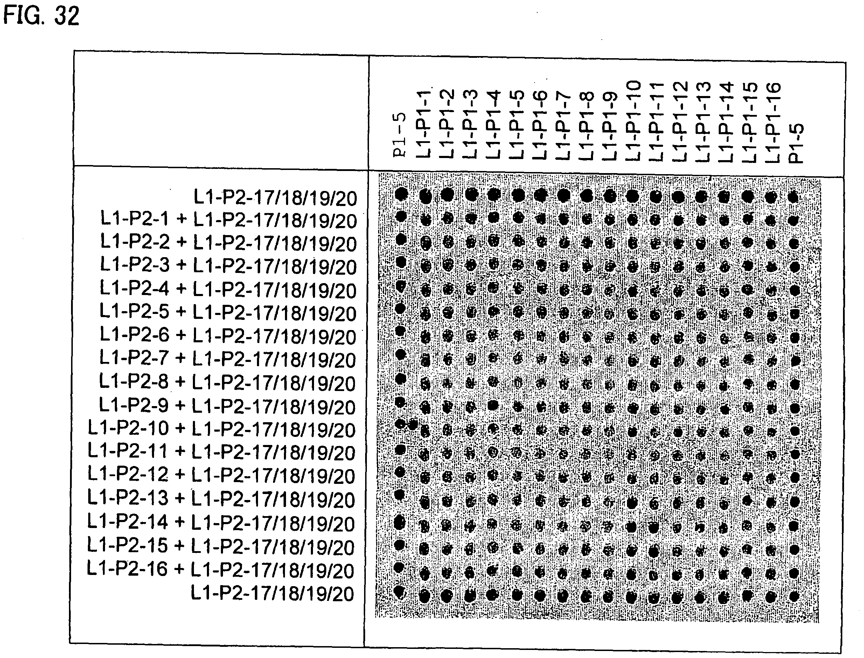

The fluorescence image for one 18x18 array of dimer and Control spots is shown in Figure 32.

Fluorescent signal is observable for each L1-P1. peptide column dispensed to the array. This indicates that each of the L1-P1 peptides has been successfully dispensed, and is capable of dimer formation. The fluorescent signal is also observable for each L1-P2 peptide row dispensed to the array. This indicates that each of the L1-P2 peptides has been successfully dispensed, and is capable of dimer formation.

The dimer fluorescence is greater for the samples with only TAMRA-labelled P2 peptides compared to the dimer fluorescence for the 16x16 array fabricated with both unlabelled P2 peptides and TAMRA-labelled P2 peptides competing for the L1-P1 peptide thiol groups. This indicates that all of the L1-P2 peptides have successfully competed with their TAMRA-labelled counterparts and have therefore successfully formed peptide dimers between all sixteen L1-P1 peptides and all sixteen L1-P2 peptides.

## Claims

1. A method of fabricating a set of dimeric peptide capture agents each of said capture agents being suitable for binding a ligand and comprising first and second peptide monomer units each comprising a ligand binding moiety and said first and second peptide monomer units being covalently linked to form a dimer, said method comprising the steps of:
a) synthesising in a combinatorial manner from first and second sets of amino acids, wherein each amino acid set is different, first and second peptide monomer units, each peptide monomer unit having a different primary amino acid sequence, each said first peptide monomer unit comprising a first reactive group and an attachment moiety comprising hydrophobic or covalent means for immobilisiing the dimeric capture agent on a substrate and each said second peptide monomer unit comprising a second reactive group; and
b) reacting said first peptide monomer units with said second peptide monomer units such that the first and second reactive groups react to form a covalent bond whereby a combinatorially-varied set of dimeric peptide capture agents is formed, wherein the combinatorial synthesis is carried out on a solid phase and prior to step (b) said first and second peptide monomer units are cleaved from the solid phase.

2. The method of claim 1 wherein the reactive groups are thiol groups.

3. The method of claim 1 which includes the further step (c) of immobilising the set of dimeric peptide capture agents on a substrate by means of the attachment moiety,
wherein step (b) is performed before, simultaneously with or subsequent to step (c).

4. The method according to claim 1, wherein the first and second peptide monomer units each comprise between 2 and 50 amino acids.

5. The method according to claim 1 or claim 2 wherein said first and second peptides include amino acids for ligand binding selected from amino acids with side chains providing a positive charge for ligand binding, amino acids providing a hydroxyl group capable of acting as a hydrogen bond donor and/or acceptor for ligand binding, amino acids providing a hydrophobic moiety for ligand binding and amino acids providing a negative charge for ligand binding.

6. The method according to claim 3 comprising covalently linking the capture agents to the substrate.

7. The method according to claim 6, comprising attaching capture agents to the substrate by native chemical ligation between thioester-derivatised capture agents and cysteine-derivatised surfaces.

8. The method according to claim 6, comprising attaching the capture agents to the substrate by native chemical ligation between capture agents with N-terminal cysteines and thioester-derivatised surfaces.

9. The method according to caim 3, comprising immobilising the capture agent on the substrate by hydrophobic interaction.

10. The method according to any one of claims 1, 2, or 9, wherein the attachment moiety is formed by each peptide comprising a plurality of hydrophobic amino acids.

11. The method according to 10, wherein the first peptide comprises a primary structure comprising alternating hydrophobic and non-hydrophobic amino acid residues.

12. The method according to any one of claims 9 to 11, comprising selecting the hydrophobic amino acids from the group consisting of leucine, isoleucine, norleucine, valine, norvaline, methionine, tyrosine, tryptophan and phenylalanine.

13. The method according to any one of claims 9 to 12, wherein the first peptide has the sequence set out in SEQ 10 No. 1.

14. The method according to any one of claims 9 to 13, wherein the second peptide has the sequence set out in SEQ ID No. 2.

## Patentansprüche

1. Verfahren zum Herstellen einer Menge dimerer Peptid-Einfangwirkstoffe, wobei jeder der Einfangwirkstoffe einen Liganden binden kann und erste und zweite Peptidraonomer-Einheiten enthält, die jeweils einen Ligandenbindungsanteil enthalten, und wobei die ersten und zweiten Peptidmonomer-Einheiten kovalent gebunden sind, um ein Dimer zu bilden, wobei das Verfahren die folgenden Schritte enthält:
a) Synthetisieren auf kombinatorische Weise aus einer ersten und einer zweiten Menge von Aminosäuren, wobei jede Ammosäurenmenge unterschiedlich ist, erster und zweiter Peptidmonomer-Einheiten, wobei jede Peptidmonomer-Einheit eine andere primäre Aminosäurmsequenz besitzt, wobei jede erste Peptidmonomer-Einheit eine erste reaktive Gruppe und einen Anlagerungsanteil, der hydrophobe oder kovalente Mittel zum Immobilisieren des dimeren Einfangwirkstoffs auf einem Substrat aufweist, enthält und jede zweite Peptidmonoiner-Einheit eine zweite reaktive Gruppe enthält; und
b) Reagierenlassen der ersten Peptidmonomer-Einheiten mit den zwei,-ten Peptidmonomer-Einheiten, so dass die erste und die zweite reaktive Gruppe reagieren, um eine kovalente Bindung zu bilden, wodurch eine kombinatorisch variierte Menge dimerer Peptid-Einfangwirkstoffe gebildet wird, wobei die kombinatorische Synthese an einer festen Phase ausgeführt wird und wobei vor dem Schritt (b) die ersten und zweiten Peptidmonomer-Einheiten von der festen Phase abgespalten werden.

2. Verfahren nach Anspruch 1, wobei die reaktiven Gruppen Thiol-Gruppen sind.

3. Verfahren nach Anspruch 1, das den weiteren Schritt (c) des Immobilisierens der Menge dimerer Peptid-Einfangwirkstoffe auf einem Substrat mittels des Anlagerungsanteils enthält,
wobei der Schritt (b) vor, gleichzeitig mit oder nach dem Schritt (c) ausgeführt wird.

4. Verfahren nach Anspruch 1, wobei die ersten und zweiten Peptidmonomer-Einheiten jeweils Aminosäuren in einer Anzahl im Bereich von 2 bis 50 enthalten.

5. Verfahren nach Anspruch 1 oder Anspruch 2, wobei die ersten und zweiten Peptide Aminosäuren für eine Ligandenbindung enthalten, die aus Aminosäuren mit Seitenketten, die eine positive Ladung für die Ligandenbindung schaffen, aus Aminosäuren, die eine Hydroxylgruppe bereitstellen, die als ein Wasserstoffbindungs-Donor und/oder als ein Wazserstoffbindurigs-Akzeptor für die Ligandenbindung wirken kann, aus Aminosäuren, die einen hydrophoben Anteil für die Ligandenbindung schaffen, und aus Aminosäuren, die eine negative Ladung für die Ligandenbindung schaffen, ausgewählt werden.

6. Verfahren nach Anspruch 3, das das kovalente Binden der Einfangwirkstoffe an das Substrat enthält.

7. Verfahren nach Anspruch 6, das das Anlagern von Einfangwixkstoffen an dem Substrat durch eine native chemische Ligation zwischen Thioester-derivatisierten Einfangwirkstoffen und Cystein-derivatisierten Oberflächen enthält.

8. Verfahren nach Anspruch 6, das das Anlagern der Einfangwirkstoffe an dem Substrat durch native chemische Ligation zwischen Einfangwirkstoffen mit N-Abschlusscysteinen und Thioester-derivatisierten Oberflächen enthält.

9. Verfahren nach Anspruch 3, das das Immobilisieren des Einfangwirkstoffs auf dem Substrat durch hydrophobe Wechselwirkung enthält.

10. Verfahren nach einem der Ansprüche 1, 2 oder 9, wobei der Anlagerungsanteil durch jedes Peptid, das mehrere hydrophobe Aminosäuren enthält, gebildet wird.

11. Verfahren nach Anspruch 10, wobei das erste Peptid eine primäre Struktur enthält, die abwechselnde hydrophobe und nicht hydrophobe Aminosäurenreste enthält.

12. Verfahren nach einem der Ansprüche 9 bis 11, das das Auszählen der hydrophoben Aminosäuren aus der Gruppe, die aus Leucin, Isoleucin, Norleucin, Valin, Normalin, Methionin, Tyrosin, Tryptophan und Phenylalanin besteht, enthält.

13. Verfahren nach einem der Ansprüche 9 bis 12, wobei das erste Peptid die Sequenz besitzt, die in SEQ ID Nr. 1 angegeben ist.

14. Verfahren nach einem der Ansprüche 9 bis 13, wobei das zweite Peptid die Sequenz besitzt, die in SEQ ID Nr. 2 angegeben ist.

## Revendications

1. Procédé pour fabriquer un ensemble d'agents de capture peptidiques dimériques, chacun desdits agents de capture étant adapté pour se lier à un ligand et comprenant une première et une seconde unité monomérique peptidique, chacune comprenant un fragment de liaison à un ligand, et lesdites première et seconde unités monomériques peptidiques étant liées de façon covalente pour former un dimère, ledit procédé comprenant les étapes consistant à :
a) synthétiser de manière combinatoire à partir d'un premier et d'un second ensemble d'acides aminés, où chaque ensemble d'acides aminés est différent, une première et une seconde unité monomérique peptidique, chaque unité monomérique peptidique ayant une séquence primaire d'acides aminés différente, chacune desdites premières unités monomériques peptidiques comprenant un premier groupe réactif et un fragment de fixation comprenant des moyens hydrophobes ou covalents pour immobiliser l'agent de capture dimérique sur un substrat, et chacune desdites secondes unités monomériques peptidiques comprenant un second groupe réactif ; et
b) faire réagir lesdites premières unités monomériques peptidiques avec lesdites secondes unités monomériques peptidiques de sorte que les premier et second groupes réactifs réagissent afin de former une liaison covalente, moyennant quoi un ensemble d'agents de capture peptidiques dimériques varié de manière combinatoire est formé, où la synthèse combinatoire est réalisée sur une phase solide et, avant l'étape (b), lesdites première et seconde unités monomériques peptidiques sont clivées de la phase solide.

2. Procédé selon la revendication 1, dans lequel les groupes réactifs sont des groupes thiols.

3. Procédé selon la revendication 1, qui comprend l'étape (c) supplémentaire consistant à immobiliser l'ensemble des agents de capture peptidiques dimériques sur un substrat au moyen du fragment de fixation,
où l'étape (b) est réalisée avant, en même temps que ou après l'étape (c).

4. Procédé selon la revendication 1, dans lequel les première et seconde unités monomériques peptidiques comprennent chacune entre 2 et 50 acides aminés.

5. Procédé selon la revendication 1 ou la revendication 2, dans lequel lesdits premier et second peptides comprennent des acides aminés pour une liaison à un ligand sélectionnés parmi des acides aminés dotés de chaînes latérales fournissant une charge positive pour une liaison à un ligand, des acides aminés fournissant un groupe hydroxyle capable d'agir en tant que donneur et/ou accepteur de liaison hydrogène pour une liaison à un ligand, des acides aminés fournissant un fragment hydrophobe pour une liaison à un ligand, et des acides aminés fournissant une charge négative pour une liaison à un ligand.

6. Procédé selon la revendication 3, qui consiste à lier les agents de capture au substrat de façon covalente.

7. Procédé selon la revendication 6, qui consiste à fixer les agents de capture au substrat à l'aide d'une ligation chimique native entre des agents de capture dérivatisés par un thioester et des surfaces dérivatisées par une cystéine.

8. Procédé selon la revendication 6, qui consiste à fixer les agents de capture au substrat à l'aide d'une ligation chimique native entre des agents de capture comportant des cystéines N-terminales et des surfaces dérivatisées par un thioester.

9. Procédé selon la revendication 3, qui consiste à immobiliser l'agent de capture sur le substrat à l'aide d'une interaction hydrophobe.

10. Procédé selon l'une quelconque des revendications 1, 2 ou 9, dans lequel le fragment de fixation est formé par chaque peptide comprenant une pluralité d'acides aminés hydrophobes.

11. Procédé selon la revendication 10, dans lequel le premier peptide comprend une structure primaire comprenant des résidus d'acides aminés hydrophobes et non hydrophobes en alternance.

12. Procédé selon l'une quelconque des revendications 9 à 11, qui consiste à sélectionner les acides aminés hydrophobes dans le groupe consistant en la leucine, l'isoleucine, la norleucine, la valine, la norvaline, la méthionine, la tyrosine, le tryptophane et la phénylalanine.

13. Procédé selon l'une quelconque des revendications 9 à 12, dans lequel le premier peptide possède la séquence décrite dans SEQ ID NO: 1.

14. Procédé selon l'une quelconque des revendications 9 à 13, dans lequel le second peptide possède la séquence décrite dans SEQ ID NO: 2.
